# EUROPEAN PATENT APPLICATION

(11) **EP 4 209 785 A1**
(43) Date of publication of application: **12.07.2023**
(21) Application number: 21863557.1
(22) Date of filing: 26.08.2021
(51) Int. Cl.: G01N 33/574, G01N 33/68

(54) **AKR1C3 DETECTION METHOD, AND DIAGNOSTIC KIT FOR DETECTING AKR1C3 AND USE THEREOF**

(30) Priority: 02.09.2020 CN 202010911697
(71) Applicant: Ascentawits Pharmaceuticals, Ltd., Shenzhen, Guangdong 518118 (CN)
(72) Inventor: XIE, Yanbin, Shenzhen, Guangdong 518118 (CN); MENG, Fanying, Shenzhen, Guangdong 518118 (CN); DUAN, Jianxin, Shenzhen, Guangdong 518118 (CN); HAO, Jing, Shenzhen, Guangdong 518118 (CN)
(74) Representative: Haseltine Lake Kempner LLP
(86) International application number: PCT/CN2021/114774
(87) International publication number: WO 2022/048492

(57) **Abstract**

An AKR1C3 detection method, and a diagnostic kit for detecting AKR1C3 and use thereof. The AKR1C3 detection method comprises the following steps: treating a formalin-fixed paraffin-embedded human tissue specimen sequentially using an organic solvent, an alcohol, and water; performing antigen retrieval on the treated formalin-fixed paraffin-embedded human tissue specimen in the presence of an antigen retrieval solution; co-incubating the antigen-retrieved formalin-fixed paraffin-embedded human tissue specimen with a blocking buffer to block a non-specific antigen; mixing the blocked formalin-fixed paraffin-embedded human tissue specimen with a certain concentration of AKR1C3 monoclonal antibody solution for primary antibody incubation, and mixing the primary antibody-incubated formalin-fixed paraffin-embedded human tissue specimen with a certain concentration of a secondary antibody solution for secondary antibody incubation. The AKR1C3 detection method can be applied to the detection of AKR1C3 expression levels in various cancer tumor tissues and is stable in dyeing results, and has good sensitivity, precision and consistency.

## Description

### Technical Field

The present invention relates to the technical field of cancer treatment, in particular to an AKR1C3 detection method, and diagnostic kit for detecting AKR1C3 and use thereof.

### Background Art

Immunohistochemical (IHC) staining method is a conventional and effective detection method for specific enzymes or proteins in pathological tissues of a patient. However, existing IHC methods are staining methods for detecting AKR1C3 expression levels which are often only developed for a single cancer tumor tissue, for example, in order to detect hepatocellular carcinoma, one IHC staining method needs to be developed separately; and in order to detect prostate cancer, another IHC staining method needs to be developed separately. In other words, the existing IHC staining detection methods cannot achieve the staining detection of AKR1C3 (aldo-keto reductase 1C3) expression levels in various cancer tissues using a single IHC assay.

In addition, since a further cancer treatment regimen needs to be determined on the basis of the IHC detection results, the IHC assay should have stable staining results, i.e., the IHC assay used in a large-scale commercial kit should have good sensitivity, precision and consistency (different laboratories, different operators, different operation times) and can be applied to various different cancer tumor tissues.

However, the staining results of IHC assay are influenced by a variety of factors (Fang Jiedi, Wang Xiaoxing, Zhang Mengling et al., Influence of water quality on immunohistochemical staining results [J]. Journal of Clinical and Experimental Pathology, 2019, 35(04):111-113; Liu Haiyang, Wang Xiaojun, Zhang Haiyu, et al., Influence of microwave heating retrieval and hydrochloric acid hydrolysis retrieval methods on immunohistochemical staining results of rat brain tissue [J]. Journal of Ningxia Medical University, 2011 (11): 1115-1116; Zhang Wei, Liang Yingjie, Influence of different antigen retrieval methods and staining conditions on the results of P53 protein immunohistochemical methods [J]. Chinese Journal of Histochemistry and Cytochemistry, 2002(2); Liu Xianyan, Yang Jian, Chen Ying, et al., Influence of different antigen retrieval solutions and retrieval methods on immunohistochemical staining results [J]. Journal of Guangdong Medical College, 2013(05):43-44; Luo Xinlan, Lin Xingtao, Luo Donglan et al., Influence of antigen retrieval solution with different components at pH 9.0 on immunohistochemical staining results [J]., Chinese Journal of Pathology, 2012, 41(003):192-194; Cai Guangling, Yu Guangyin, Zhao Yang., Influence of pH of antigen retrieval solution on immunohistochemical staining of lymphoid tissues [J], Acta Medicinae Sinica, 2005, 18(4):501-502; Du Juan, Shi Xueying, Zheng Jie et al., Influence of pH and retrieval time of antigen retrieval solution on the immunohistochemical staining effects [J], Journal of Peking University (Health Sciences), 2005, 037(002):195-197; and Meng Kui, Zhou Xiaojun, The role of antigen retrieval techniques in immunohistochemistry [J], Chinese Journal of Histochemistry and Cytochemistry, 2001, 10(001):109-111.), the influencing processes include the antigen retrieval process, the staining process, etc., and the influencing factors and conditions include the heating temperature, the heating time, the water quality used, the composition of the antigen retrieval solution, the pH of the antigen retrieval solution and the time of the retrieval reaction, etc., and certainly the different tissue types will greatly affect the results of the IHC staining detection. These factors result in the instability of the staining results of the IHC assay disclosed in the prior art, i.e., the lack of good sensitivity, precision and consistency, and the IHC assay disclosed in the prior art cannot be applied to the detection of many different types of cancers. Therefore, there is a need to develop an AKR1C3 detection method, which can be applied to the detection of AKR1C3 expression levels in various cancer tumor tissues and is stable in staining results.

### Summary of the Invention

In view of the above, the object of the present invention is to provide an AKR1C3 detection method, a diagnostic kit for detecting AKR1C3 and use thereof, wherein the AKR1C3 detection method and the kit can be applied to the detection of AKR1C3 expression levels in various cancer tumor tissues and is stable in staining results, and has good sensitivity, precision and consistency.

Based on the above object, one aspect of the present invention provides an AKR1C3 detection method, wherein the AKR1C3 expression levels in isolated formalin-fixed paraffin-embedded human tissue specimen is detected by using immunohistochemical staining method, comprising the following steps:
a) antigen retrieval
   performing antigen retrieval by heating the formalin-fixed paraffin-embedded human tissue specimen at 90~115°C for 17-30 min in the presence of an antigen retrieval solution;
b) primary antibody incubation mixing the antigen-retrieved formalin-fixed paraffin-embedded human tissue specimen with a 0.5~5.0 µg/ml concentration of AKR1C3 monoclonal antibody solution for incubation for 25-700 min;
c) secondary antibody incubation mixing the primary antibody-incubated formalin-fixed paraffin-embedded human tissue specimen with a 0.5~5.0 µg/ml concentration of secondary antibody solution for incubation for 25-700 min.

In a preferred embodiment of the present invention, in the antigen retrieval of step a), the antigen retrieval solution has a pH of 2.0 ~ 9.0; more preferably, the antigen retrieval solution has a pH of 6.0 ~ 9.0; even more preferably, the antigen retrieval solution has a pH of 6.0.

In a preferred embodiment of the present invention, in the antigen retrieval of step a), the antigen retrieval solution includes sodium citrate antigen retrieval solution or EDTA antigen retrieval solution.

In a preferred embodiment of the present invention, in the antigen retrieval of step a), the formalin-fixed paraffin-embedded human tissue specimen is heated at 92-102 °C for 18-25 min;
more preferably, the formalin-fixed paraffin-embedded human tissue specimen is heated at 97 °C for 20 min.

In a preferred embodiment of the present invention, in the primary antibody incubation of step b), the AKR1C3 monoclonal antibody solution has a concentration of 1.0~3.0 µg/ml;
more preferably, the AKR1C3 monoclonal antibody solution has a concentration of 1.2 µg/ml;
and/or, in the secondary antibody incubation of step c), the secondary antibody solution has a concentration of 1.0~3.0 µg/ml;
more preferably, the secondary antibody solution has a concentration of 1.2 µg/ml.

In a preferred embodiment of the present invention, the AKR1C3 monoclonal antibody solution and the secondary antibody solution both contain NaN₃, H⁺, Cl⁻ and tromethamine.

In a preferred embodiment of the present invention, the AKR1C3 monoclonal antibody solution and the secondary antibody solution are obtained by diluting with an antibody dilution buffer, wherein the antibody dilution buffer comprises the following components:
0.02~0.08 mol/L of Tris-HCl buffer,
containing 0.05~0.15% mass concentration of polyethylene glycol or Tween, and
0.010~0.020 mol/L of sodium azide;
more preferably, the antibody dilution buffer comprises the following components:
0.05 mol/L of Tris-HCl buffer,
containing 0.1% mass concentration of polyethylene glycol or Tween, and 0.015 mol/L of sodium azide.

In a preferred embodiment of the present invention, in the primary antibody incubation of step b), the antigen-retrieved formalin-fixed paraffin-embedded human tissue specimen is incubated with the AKR1C3 monoclonal antibody solution for 30-45 min;
more preferably, the antigen-retrieved formalin-fixed paraffin-embedded human tissue specimen is incubated with the AKR1C3 monoclonal antibody solution for 45 min.

In a preferred embodiment of the present invention, in the secondary antibody incubation of step c), the primary antibody-incubated formalin-fixed paraffin-embedded human tissue specimen is incubated with the secondary antibody solution for 30-45 min;
more preferably, the primary antibody-incubated formalin-fixed paraffin-embedded human tissue specimen is incubated with the secondary antibody solution for 30 min.

In a preferred embodiment of the present invention, in the primary antibody incubation of step b), the AKR1C3 monoclonal antibody is a mouse monoclonal antibody;
and/or, in the secondary antibody incubation of step c), the secondary antibody is a goat anti-mouse antibody, a rabbit anti-mouse antibody, a horse anti-mouse antibody or a donkey anti-mouse antibody.

In a preferred embodiment of the present invention, after the secondary antibody incubation of step c), further comprising:
d) staining and sealing
staining the formalin-fixed paraffin-embedded human tissue specimen using hematoxylin, and performing the dehydration and sealing of specimen after staining.

In a preferred embodiment of the present invention, before the antigen retrieval of step a), further comprising:
a1) dewaxing and rehydration
dewaxing the formalin-fixed paraffin-embedded human tissue specimen using organic solvent, washing the dewaxed specimen sequentially using alcohols containing different water contents, and finally washing with water;
preferably, the organic solvent is acetone, toluene or xylene; more preferably, the organic solvent is xylene;
and/or, preferably, the alcohol is ethanol or methanol; more preferably, the alcohol is ethanol;
and/or, preferably, the dewaxed specimen is first washed with anhydrous ethanol and then washed using ethanol having a volume fraction of 90~97%.

In a preferred embodiment of the present invention, between the antigen retrieval of step a) and the primary antibody incubation of step b), further comprising:
b1) blocking non-specific antigen
co-incubating the antigen-retrieved formalin-fixed paraffin-embedded human tissue specimen with a blocking solution to block a non-specific antigen;
preferably, the blocking solution is a serum of an animal from which the AKR1C3 monoclonal antibody is derived;
more preferably, the blocking solution is mouse serum.

In a preferred embodiment of the invention, the formalin-fixed paraffin-embedded human tissue specimen is breast cancer tissue specimen, colorectal cancer tissue specimen, esophageal cancer tissue specimen, gastric cancer tissue specimen, hepatocellular carcinoma tissue specimen, non-small cell lung cancer tissue specimen, prostate cancer tissue specimen, renal cell carcinoma specimen, peripheral T-cell lymphoma specimen or nodular NK/T-cell lymphoma specimen.

Based on the same inventive concept, another aspect of the present invention provides a diagnostic kit for detecting AKR1C3, comprising:
antigen retrieval solution;
a 0.5~5.0 µg/ml concentration of AKR1C3 monoclonal antibody solution;
a 0.5~5.0 µg/ml concentration of secondary antibody solution.

In a preferred embodiment of the present invention, the antigen retrieval solution has a pH of 2.0 ~ 9.0;
more preferably, the antigen retrieval solution has a pH of 6.0 ~ 9.0;
even more preferably, the antigen retrieval solution has a pH of 6.0.

In a preferred embodiment of the present invention, the antigen retrieval solution includes sodium citrate antigen retrieval solution or EDTA antigen retrieval solution.

In a preferred embodiment of the present invention, the AKR1C3 monoclonal antibody solution has a concentration of 1.0~3.0 µg/ml;
more preferably, the AKR1C3 monoclonal antibody solution has a concentration of 1.2 µg /ml;
and/or, the secondary antibody solution has a concentration of 1.0~3.0 µg/ml;
more preferably, the secondary antibody solution has a concentration of 1.2 µg/ml.

In a preferred embodiment of the present invention, the AKR1C3 monoclonal antibody solution and the secondary antibody solution both contain NaN₃, H⁺, Cl⁻ and tromethamine.

In a preferred embodiment of the present invention, the AKR1C3 monoclonal antibody solution and the secondary antibody solution are obtained by diluting with an antibody dilution buffer, wherein the antibody dilution buffer comprises the following components:
0.02~0.08 mol/L of Tris-HCl buffer,
containing 0.05~0.15% mass concentration of polyethylene glycol or Tween, and
0.010~0.020 mol/L of sodium azide;
more preferably, the antibody dilution buffer comprises the following components:
0.05 mol/L of Tris-HCl buffer,
containing 0.1% mass concentration of polyethylene glycol or Tween, and 0.015 mol/L of sodium azide.

In a preferred embodiment of the present invention, the AKR1C3 monoclonal antibody is a mouse monoclonal antibody;
and/or, the secondary antibody is a goat anti-mouse antibody, a rabbit anti-mouse antibody, a horse anti-mouse antibody or a donkey anti-mouse antibody.

In a preferred embodiment of the present invention, the above diagnostic kit for detecting AKR1C3 further comprises:
blocking solution, preferably the blocking solution is a serum of an animal from which the AKR1C3 monoclonal antibody is derived;
more preferably, the blocking solution is mouse serum.

In a preferred embodiment of the present invention, the diagnostic kit for detecting AKR1C3 further comprises:
negative control reagent solution; and
instructions.

Based on the same inventive concept, another aspect of the present invention provides use of the above diagnostic kit for detecting AKR1C3 in the preparation of drugs for the treatment of cancer, tumor or cell proliferative disease.

In a preferred embodiment of the present invention, the use described above comprises the following steps:
obtaining AKR1C3 expression levels in isolated formalin-fixed paraffin-embedded human tissue specimen from a patient using the diagnostic kit for detecting AKR1C3 as described above;
administering AKR1C3-activated anticancer drug to the patient whose AKR1C3 expression levels are greater than or equal to predetermined expression levels.

In a preferred embodiment of the invention, the AKR1C3-activated anticancer drug meets but is not limited to at least one of the following definitions:
A. in the presence of an AKR1C3 inhibitor, the inhibition effect detected of a compound on the proliferation of cancer cells is less than that of cancer cells in the absence of an AKR1C3 inhibitor;
B. the inhibition effect of a compound on the proliferation of cancer cells with different expression levels of AKR1C3 enzyme is significantly different, and the inhibition effect on the proliferation of cancer cells with high AKR1C3 enzyme expression is much greater than that of cancer cells with low AKR1C3 enzyme expression;
C. when the inhibition effect of certain aldehyde-ketone compound containing carbon-oxygen double bond on the proliferation of cancer cells with different expression levels of AKR1C3 enzyme is significantly different, and the inhibition effect on the proliferation of cancer cells with high AKR1C3 enzyme expression is much greater than that of cancer cells with low AKR1C3 enzyme expression, and the difference among the inhibition effects of the corresponding alcohol compound containing hydroxyl group on the proliferation of cancer cells with different expression levels of AKR1C3 enzyme is small or similar, then the aldehyde-ketone compound containing carbon-oxygen double bond is AKR1C3-activated anticancer drug, and the corresponding alcohol compound containing hydroxyl group is parent drug.

In a preferred embodiment of the present invention, the AKR1C3-activated anticancer drug is selected from the compounds of the following structures: 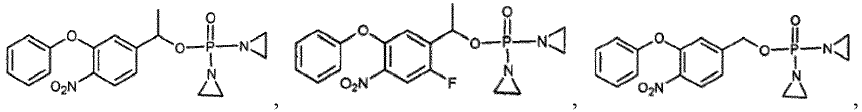 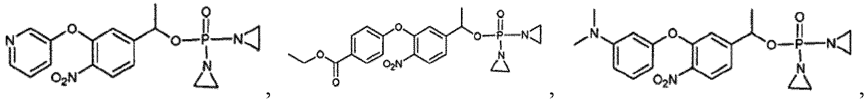 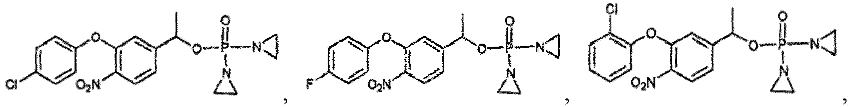 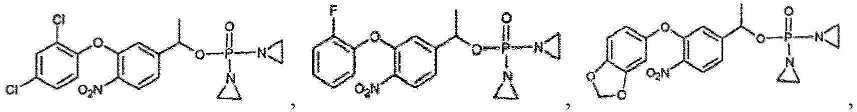 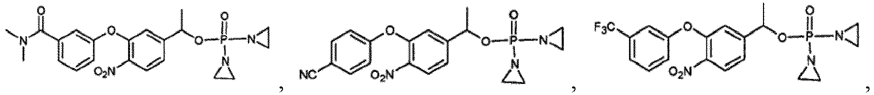 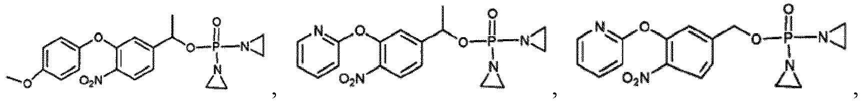 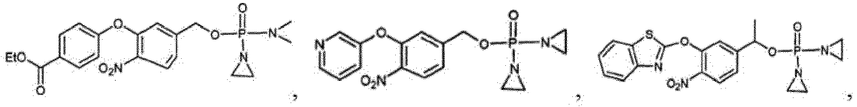 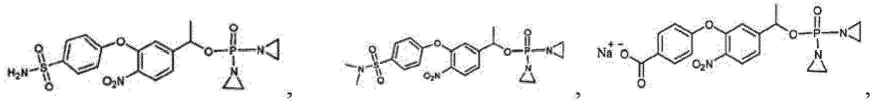 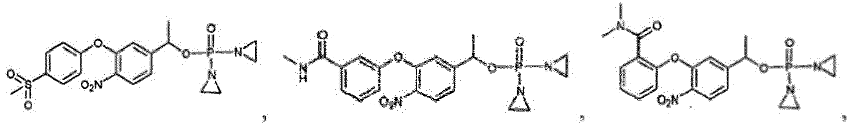 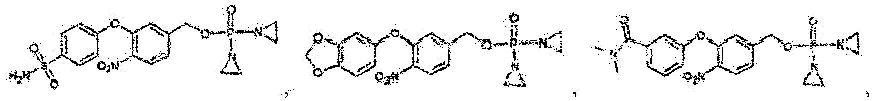 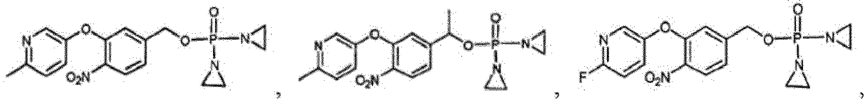 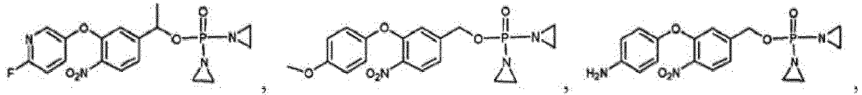 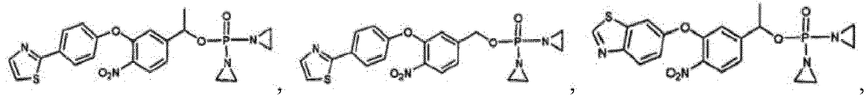 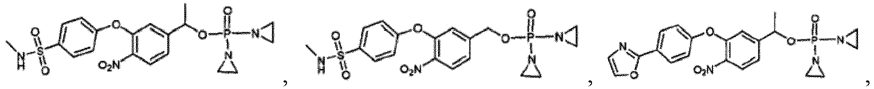 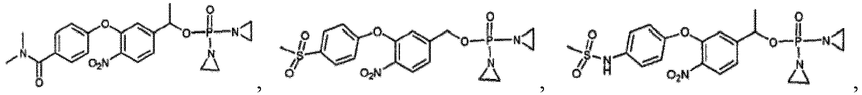 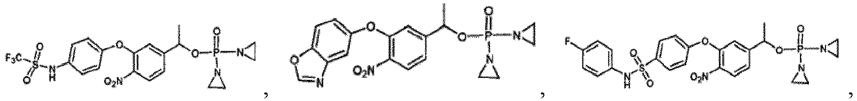 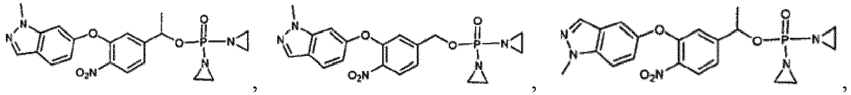 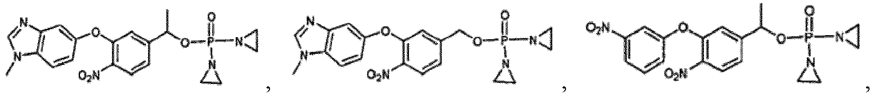 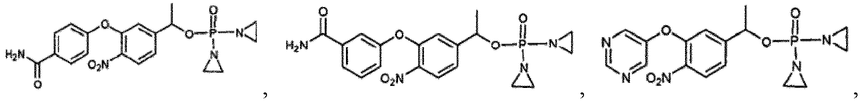 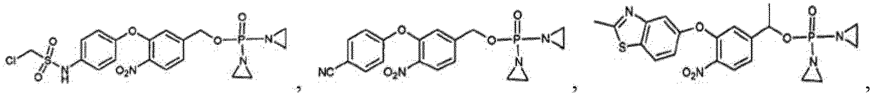 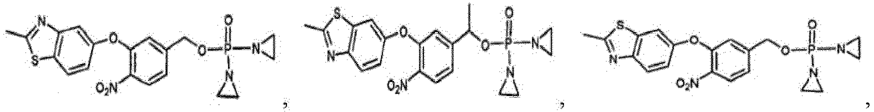 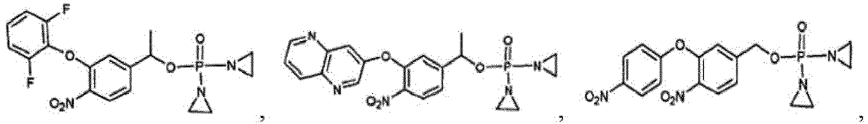 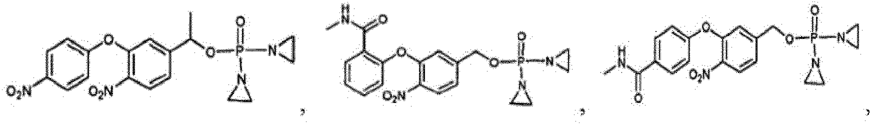 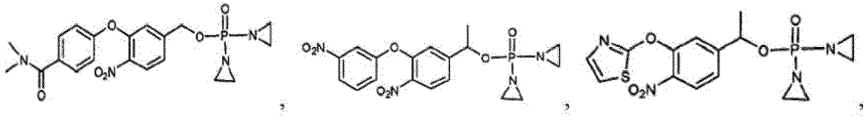 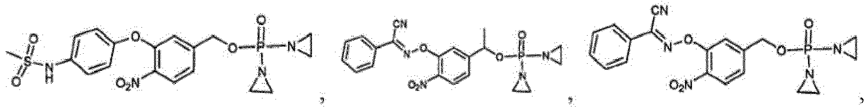 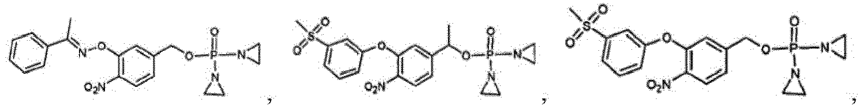 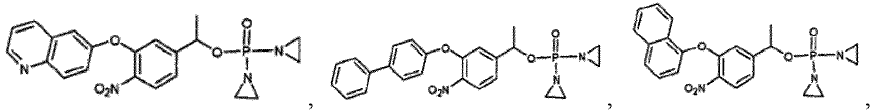 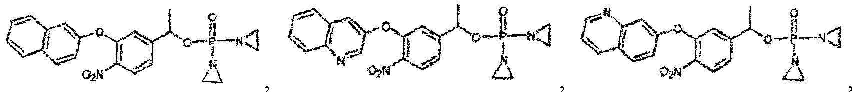 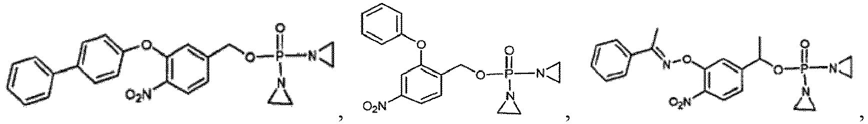 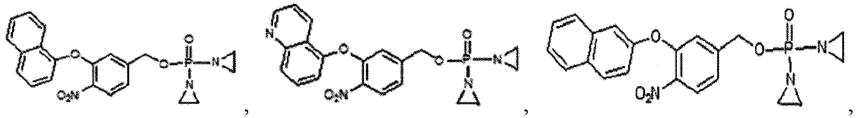 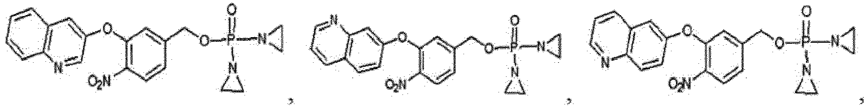 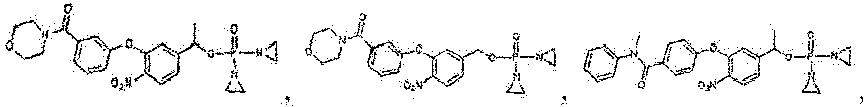 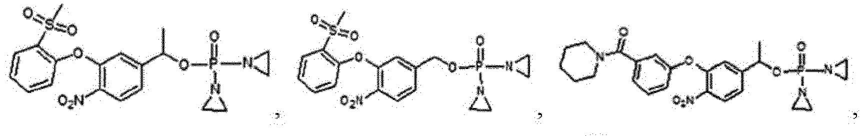 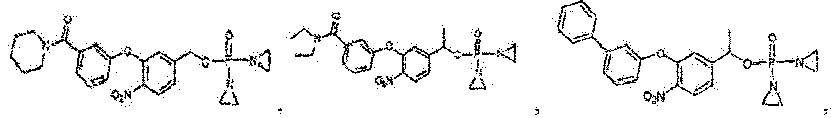 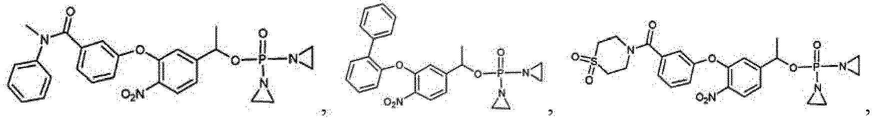 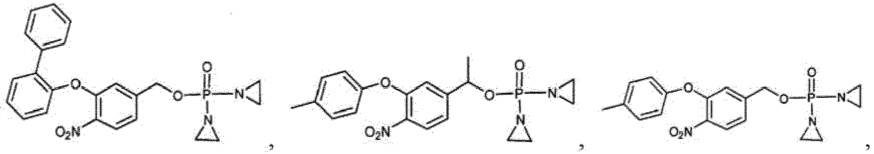 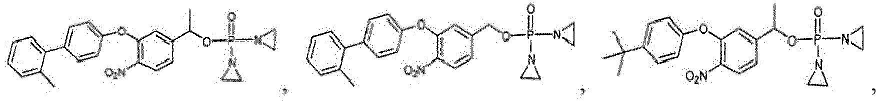 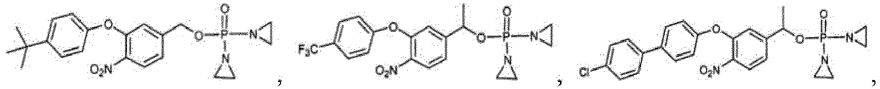 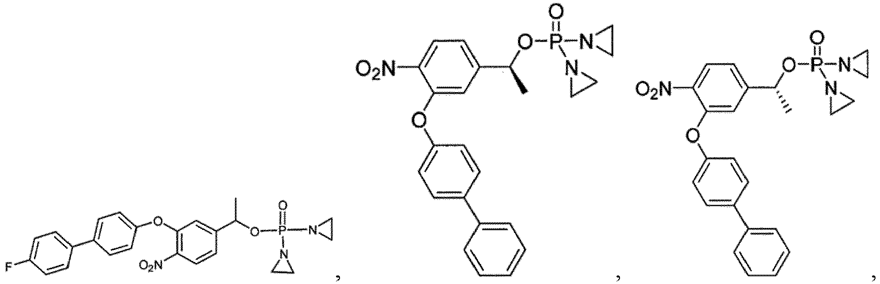 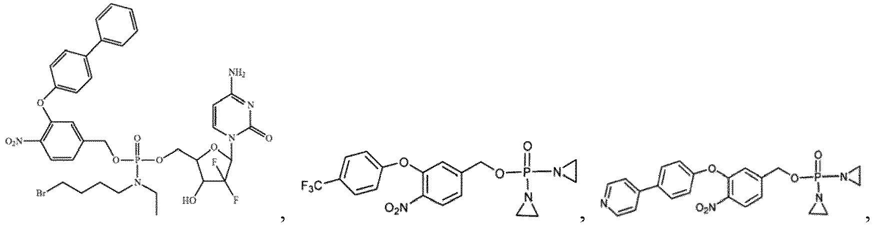 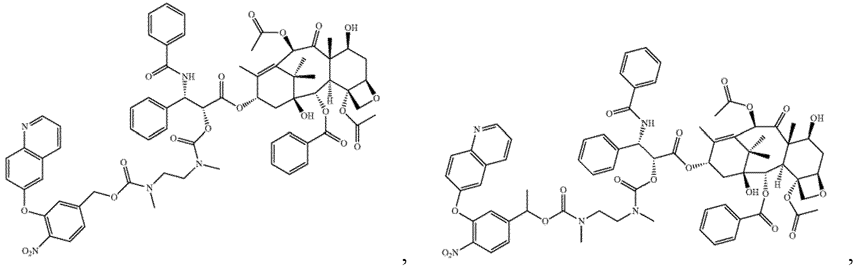 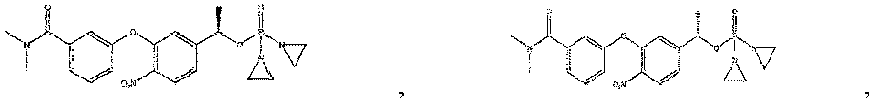 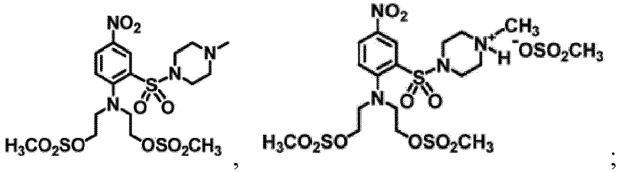 or
the compounds 5-nitrobenzenesulfonamide dibromide, bromomethylsulfonate and bis(methylsulfonyl)mustard (Compound 562-674), 3-methyl-5-nitrobenzenesulfonamide dibromide, bromomethylsulfonate and bis(methylsulfonyl)mustard (Compound 679-791), 3-trifluoromethyl-5-nitrobenzenesulfonamide dibromide, bromomethylsulfonate and bis(methylsulfonyl)mustard (Compound 913-1025), 3-ethynyl-5-nitrobenzenesulfonamide dibromide, bromomethylsulfonate and bis(methylsulfonyl)mustard (Compound 1030-1142), and methanesulfonate of 5-nitrobenzenesulfonamide bis(methylsulfonyl) mustard (compounds 640. Ms, 641. Ms, 642. Ms, 643. Ms, 644. Ms, 757. Ms, 758. Ms, 991. Ms, 992. Ms, 1108. Ms and 1109. Ms) in Patent PCT/NZ2019/050030, with Publication No. WO2019190331; or 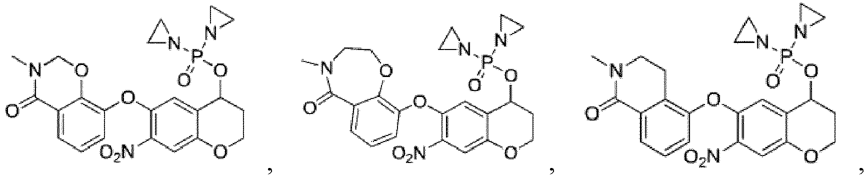 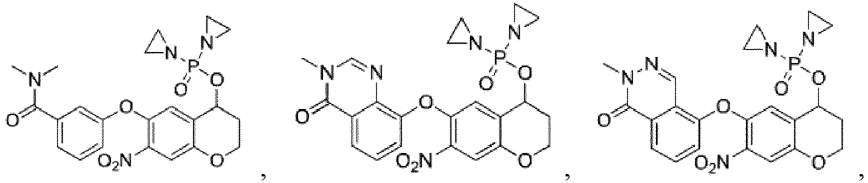 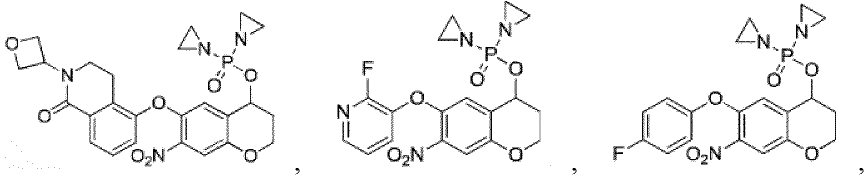 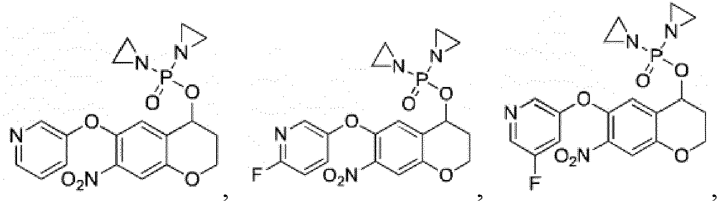 or a pharmaceutically acceptable salt or isomer thereof; or
Compounds Ex1 to Ex170 in Patent PCT/IB2020/057285, with Publication No. WO2021005586A1; or 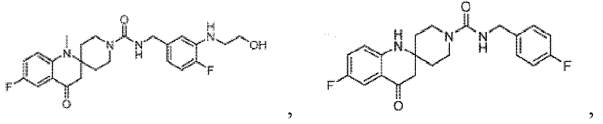 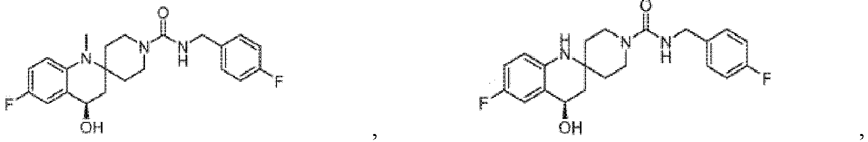 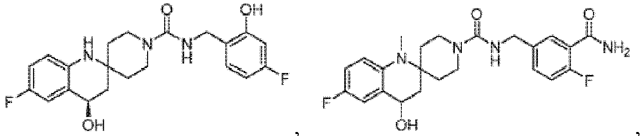 or a pharmaceutically acceptable salt or isomer thereof;

Preferably, the AKR1C3-activated anticancer drug is selected from the compounds of the following structures: 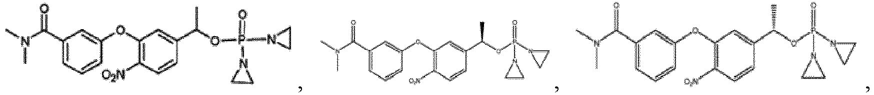 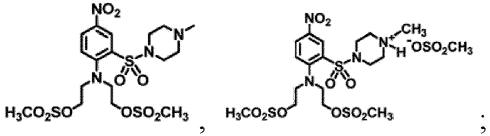 or 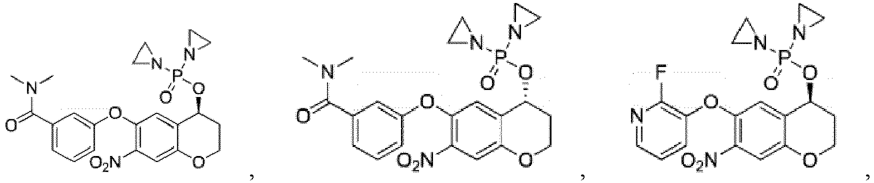 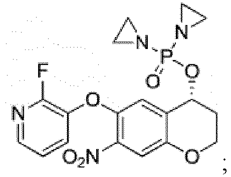 or 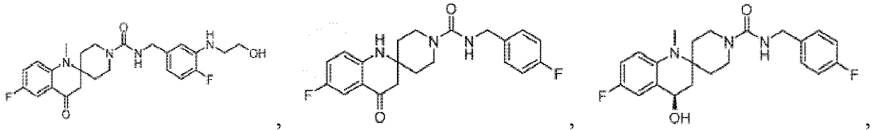 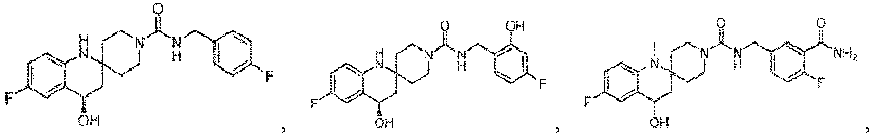 or a pharmaceutically acceptable salt or isomer thereof.

In a preferred embodiment of the invention, the cancer, tumor or cell proliferative disease comprises:
lung cancer, non-small cell lung cancer, liver cancer, pancreatic cancer, breast cancer, gastric cancer, bone cancer, esophageal cancer, mastocarcinoma, prostate cancer, testicular cancer, colon cancer, ovarian cancer, bladder cancer, cervical cancer, hepatocellular carcinoma, melanoma, squamous cell carcinoma, basal cell carcinoma, adenocarcinoma, sweat gland carcinoma, sebaceous gland carcinoma, papillary carcinoma, papillary adenocarcinoma, renal cell carcinoma, cystic adenocarcinoma, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, peripheral T-cell lymphoma, nodular NK/T-cell lymphoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, chronic disease cells, polycythemia, lymphoma, endometrial cancer, glioma, colorectal cancer, thyroid cancer, urothelial cancer or multiple myeloma;
preferably, the cancer, tumour or cell proliferative disease comprises: ovarian cancer, cervical cancer, pancreatic cancer, breast cancer, colorectal cancer, esophageal cancer, gastric cancer, hepatocellular carcinoma, non-small cell lung cancer, prostate cancer, renal cell carcinoma, peripheral T-cell lymphoma or nodular NK/T-cell lymphoma.

### Brief Description of The Drawings

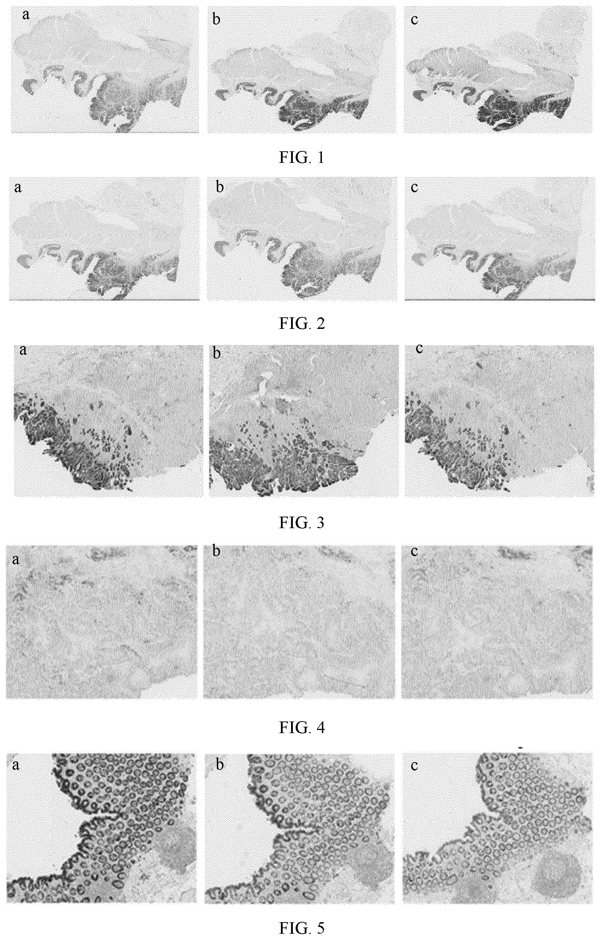

FIG. 1 shows the IHC staining photographs of CRC samples after retrieval with high pH of antigen retrieval solution, Scanscope scan 0.4x, CRC sample: 335933-P; wherein the figure a corresponds to Ab (antibody): 1:1000, incubation time for primary antibody and secondary antibody is 20 min and 20 min respectively; the figure b corresponds to Ab: 1:2000, incubation time for primary antibody and secondary antibody is 45 min and 30 min respectively; and the figure c corresponds to Ab: 1:2000, incubation time for primary antibody and secondary antibody is 60 min and 30 min respectively;
FIG. 2 shows the IHC staining photographs of CRC samples after retrieval with low pH of antigen retrieval solution, Scanscope scan 0.4x, CRC sample: 335933-P; wherein the figure a corresponds to Ab: 1:1000, incubation time for primary antibody and secondary antibody is 30 min and 30 min respectively; the figure b corresponds to Ab: 1:2000, incubation time for primary antibody and secondary antibody is 45 min and 30 min respectively; and the figure c corresponds to Ab: 1:2000, incubation time for primary antibody and secondary antibody is 60 min and 30 min respectively;
FIG. 3 shows photographs of the IHC staining results of gastric cancer samples at low pH; wherein the figure a corresponds to Ab: 1:1000, incubation time for primary antibody and secondary antibody is 30 min and 30 min respectively; the figure b corresponds to Ab: 1:2000, incubation time for primary antibody and secondary antibody is 45 min and 30 min respectively; and the figure c corresponds to Ab: 1:2000, incubation time for primary antibody and secondary antibody is 45 min and 45 min respectively;
FIG. 4 shows photographs of the IHC staining results of breast cancer samples at low pH; wherein the figure a corresponds to Ab: 1:1000, incubation time for primary antibody and secondary antibody is 30 min and 30 min respectively; the figure b corresponds to Ab: 1:2000, incubation time for primary antibody and secondary antibody is 45 min and 30 min respectively; the figure c corresponds to Ab: 1:2000, incubation time for primary antibody and secondary antibody is 45 min and 45 min respectively;
FIG. 5 shows photographs of the staining performance comparison under different monoclonal antibody dilutions and incubation times, taking normal colon tissue: 391761-YN as an example, Scanscope scan 4x; wherein the figure a corresponds to Ab: 1:1000, incubation time for primary antibody and secondary antibody is 30 min and 30 min respectively; the figure b corresponds to Ab: 1:2000, incubation time for primary antibody and secondary antibody is 45 min and 30 min respectively; the figure c corresponds to Ab: 1:2000, incubation time for primary antibody and secondary antibody is 30 min and 30 min respectively;

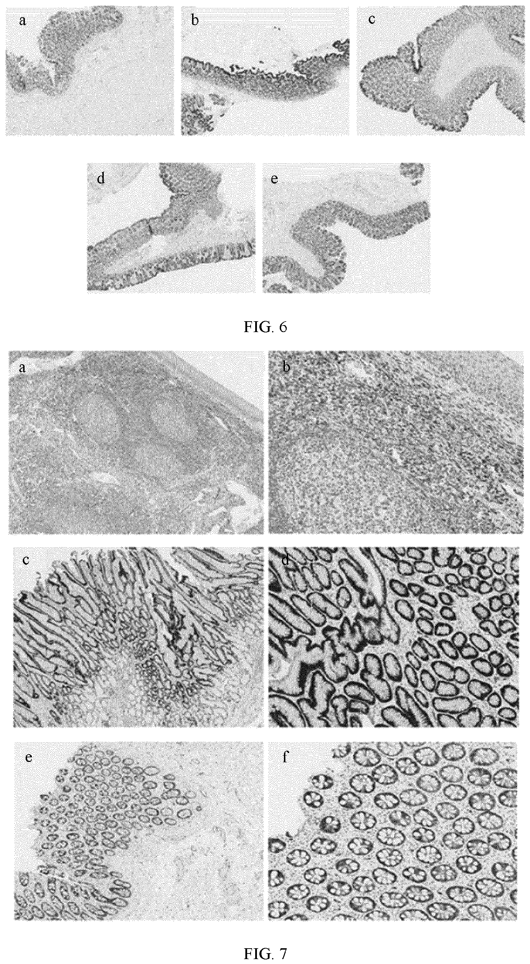

FIG. 6 shows the comparison of the staining performance of consistency between five different normal colon tissues using the optimal staining protocol: low pH TRS, 97°C 20 min; AKR1C3 dilution 1:2000, 30 min; HRP incubation time: 30 min, Scanscope scan 2x; wherein figure a corresponds to sample 390211-YN, figure b corresponds to sample 390650-YN, figure c corresponds to sample 391182-YN, figure d corresponds to sample 391761-YN and figure e corresponds to sample 3919951-YN;
FIG. 7 shows photographs of AKR1C3 staining in normal tissues using the optimal staining conditions; wherein figure a and figure b correspond to low and high magnification staining photographs of normal tonsil tissues, respectively; figure c and figure d correspond to low and high magnification staining photographs of normal stomach tissues, respectively; and figure e and figure f correspond to low and high magnification staining photographs of normal colon tissues, respectively;

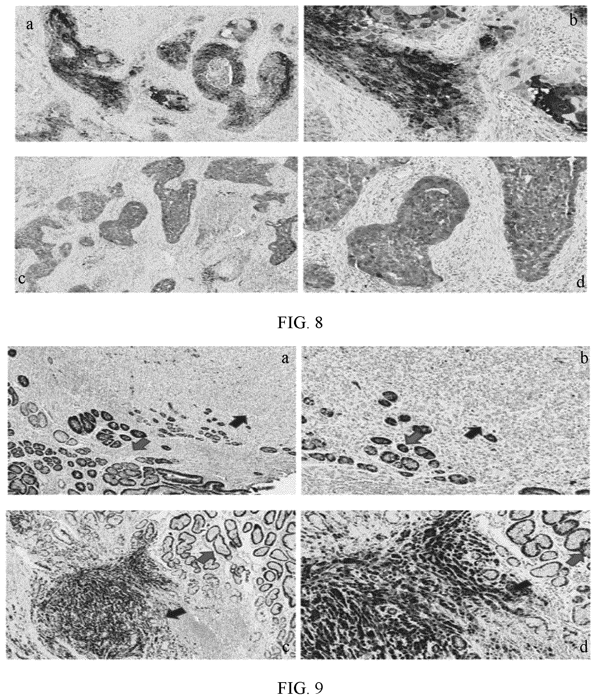

FIG. 8 shows photographs of AKR1C3 staining in non-small cell lung cancer using the optimal protocol; wherein figure a and figure b correspond to low and high magnification staining photographs of sample 1 (F102582A22), respectively; and figure c and figure d correspond to low and high magnification staining photographs of sample 2 (F134064A12), respectively;
FIG. 9 shows photographs of AKR1C3 staining in gastric cancer using the optimal protocol; wherein figure a and figure b correspond to low and high magnification staining photographs of sample 1 (F180723A5), respectively, up arrow: tumour cells, down arrow: remaining normal gastric mucosal glandular epithelium; and figure c and figure d correspond to low and high magnification staining photographs of sample 2 (F180684A3), respectively, down arrow: tumour cells, up arrow: remaining normal gastric mucosal glandular epithelium;

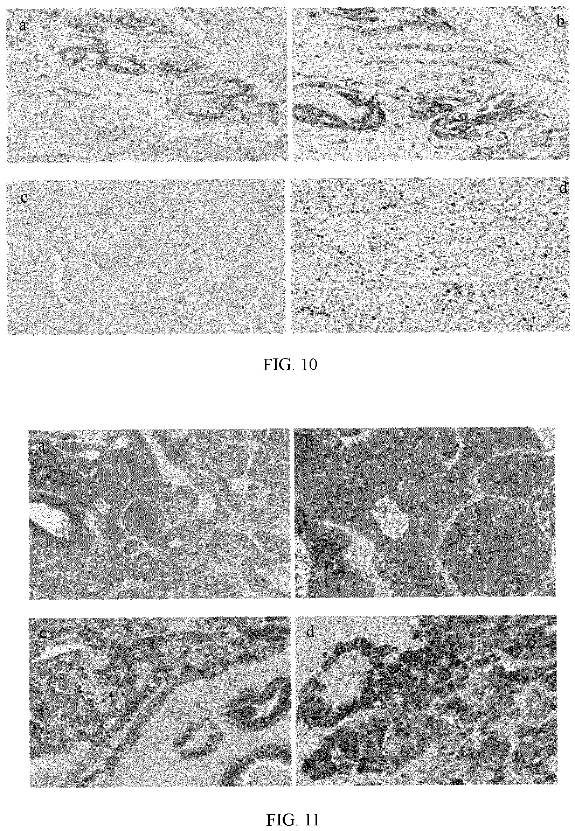

FIG. 10 shows photographs of AKR1C3 staining in breast cancer using the optimal protocol; wherein figure a and figure b correspond to low and high magnification staining photographs of sample 1 (F162870A5), respectively; and figure c and figure d correspond to low and high magnification staining photographs of sample 2 (F130368B3), respectively;
FIG. 11 shows photographs of AKR1C3 staining in hepatocellular carcinoma using the optimal protocol; wherein figure a and figure b correspond to low and high magnification staining photographs of sample 1 (DLV13050B3), respectively; and figure c and figure d correspond to low and high magnification staining photographs of sample 2 (DLV13052B5), respectively;

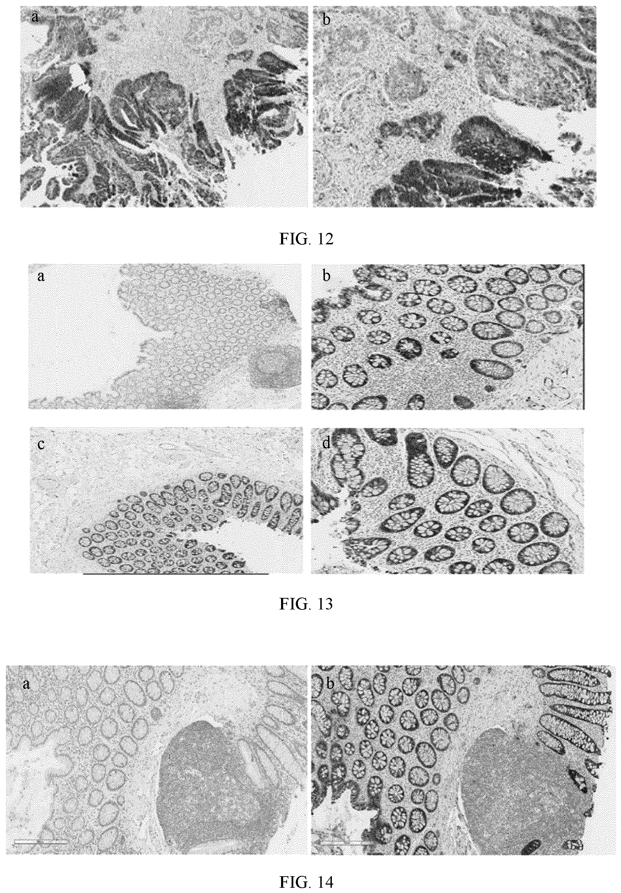

FIG. 12 shows photographs of AKR1C3 staining in colorectal cancer using the optimal protocol; wherein figure a and figure b correspond to low and high magnification staining photographs of sample (335933-P), respectively;
FIG. 13 shows photographs of staining in normal colon tissue with both positive and negative components under the optimal protocol of AKR1C3 IHC assay, scanned at different magnifications; wherein figure a corresponds to a negative control reagent (Scanscope scan 4x), figure b corresponds to AKR1C3 (Scanscope scan 10x), figure c corresponds to AKR1C3 (Scanscope scan 4x) and figure d corresponds to AKR1C3 (Scanscope scan 10x);
FIG. 14 shows photographs of tissue quality control samples, where normal colon tissue with both positive and negative components was used as a double positive and negative tissue quality control and for each staining operation; wherein figure a corresponds to the negative control reagent (Scanscope scan 4x) and figure b corresponds to AKR1C3 staining (Scanscope scan 4x);

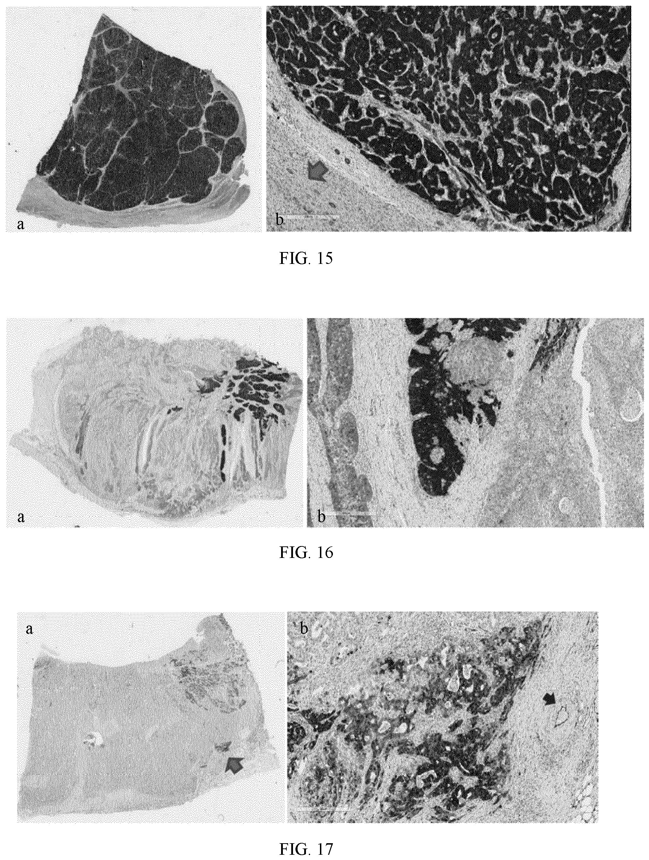

FIG. 15 shows a photograph of sample F151286A5 HCC with an H-score of 300 (3+: 100%). All tumour cells show strong cytoplasm/nucleus staining; meanwhile, normal hepatocytes near the cancer nests (arrows) as well as stromal cells and endothelial cells showing different intensities of staining as internal quality control; wherein figure a corresponds to a lower magnification times (Scanscope scan 0.4x) and figure b corresponds to a higher magnification times (Scanscope scan 10x);
FIG. 16 shows a photograph of sample F151725A1 EC with an H-score of 160 (0: 0%; 1+: 60%; 2+: 20%; 3+: 20%); the tumour cells show different intensities of different cytoplasm/nucleus staining; wherein figure a corresponds to a lower magnification times (Scanscope scan 0.4x) and figure b corresponds to a higher magnification times (Scanscope scan 10x);
FIG. 17 shows a photograph of sample F152459A4 GC with an H-score of 35 (0: 85%; 1+: 5%; 2+: 0%; 3+: 10%); the tumour cells show different intensities of different cytoplasm/nucleus staining. The higher the magnification times, the smaller the area shown (up arrow); endothelial cells showing staining as internal quality control (down arrow); wherein figure a corresponds to a lower magnification times (Scanscope scan 0.4x) and figure b corresponds to a higher magnification times (Scanscope scan 10x);

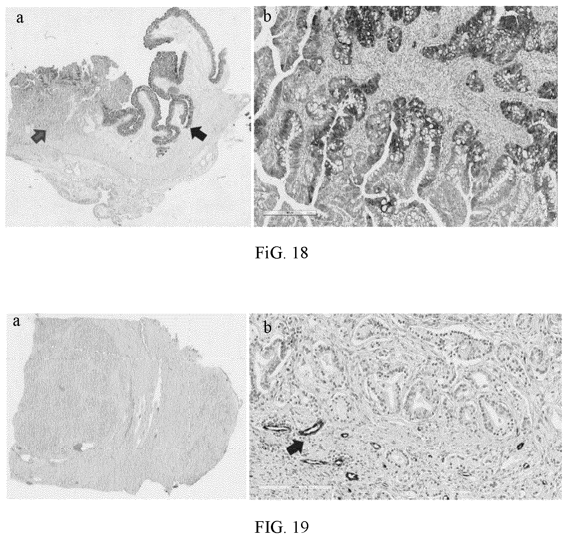

FIG. 18 shows a photograph of sample F151653A1 CRC with an H-score of 120 (0: 30%; 1+: 30%; 2+: 30%; 3+: 10%); the tumour cells show different intensities of different cytoplasm/nucleus staining; the tumour area is shown with a blue arrow to the right and the normal tissue is shown with a arrow to the left; wherein figure a corresponds to a lower magnification times (Scanscope scan 0.4x) and figure b corresponds to a higher magnification times (Scanscope scan 10x);
FIG. 19 shows a photograph of sample F183410A4 PC with an H-score of 0 (0: 100%); no tumour cells show cytoplasm/nucleus staining for AKR1C3; however, endothelial cells showing staining as an internal quality control (arrow); wherein figure a corresponds to a lower magnification times (Scanscope scan 0.4x) and figure b corresponds to a higher magnification times (Scanscope scan 10x).

### Detailed Description of the Invention

It is necessary to indicate that, unless otherwise defined, technical terms or scientific terms used in one or more examples of the present specification shall have the ordinary meaning as understood by people having ordinary skill in the field to which the present disclosure belongs.

The experimental methods in the following examples are all conventional methods unless otherwise specified. The raw materials of the medicaments, the reagents and the like used in the following examples are all commercially available products unless otherwise specified.

"Patient" and "subject" are used interchangeably herein and refer to a mammal in need of treatment for cancer. Generally, the patient is a human. Generally, the patient is a human diagnosed with cancer. In certain examples, a "patient" or "subject" may refer to a non-human mammal used in screening, characterizing, and evaluating drugs and therapies, such as, a non-human primate, a dog, cat, rabbit, pig, mouse or a rat.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392, incorporated herein by reference). A prodrug may be synthesized using reactants other than the corresponding drug.

"Treatment of" a condition or patient refers to taking steps to obtain beneficial or desired results, including clinical results. For purposes of this invention, beneficial or desired clinical results include, but are not limited to, alleviation or improvement of one or more symptoms of cancer; diminishment of extent of disease; delay or slowing of disease progression; alleviation, palliation, or stabilization of the disease state; or other beneficial results. Treatment of cancer may, in some cases, result in partial response or stable disease.

"Tumor cells" refers to tumor cells of any appropriate species, e.g., mammalian such as murine, canine, feline, equine or human.

DNA alkylating agents of anticancer prodrugs targeting over-expression of AKR1C3 developed by the applicant of the present invention include: 1) DNA alkylating agent, corresponding to PCT Application No. PCT/US2016/021581, Publication No. WO2016/145092A, corresponding to Chinese Application No. 2016800150788, Publication No. CN107530556A; 2) (R)- and (S)-1-(3-(3-N,N-dimethylaminocarbonyl)phenoxyl-4-nitrophenyl)-1-ethyl-N,N'-bis(ethylene)phosphoramidate, compositions and methods for their use and preparation, corresponding to PCT Application No. PCT/US2016/062114, Publication No. WO2017087428A1, corresponding to Chinese Application No. 2016800446081, Publication No. CN108290911A; 3) Nitrobenzyl derivatives of anticancer reagents, corresponding to PCT Application No. PCT/US2016/025665, Publication No. WO2016/161342, corresponding to Chinese Application No. 2016800200132, Publication No. CN108136214A. The compounds in the form of prodrugs are reduced under the catalysis of AKR1C3 in the biochemical environment in the cells to obtain cytotoxic toxins, thereby exerting toxic effect on cancer cells.

In particular, the S-configuration compound with the name of (S)-1-(3-(3-N,N-dimethylaminocarbonyl)phenoxyl-4-nitrophenyl)-1-ethyl-N,N'-bis(et hylidene)phosphoramidate (also referred to as OBI-3424, AST-3424, TH-2870), is shown as CAS No. 2097713-69-2, which has the following structure: 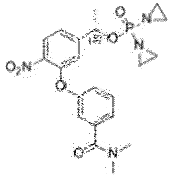 which has been in Phase I clinical trials in the U.S. and China, respectively.

The above drugs is only effective in the patients with AKR1C3 expression, so it is necessary to detect the AKR1C3 expression level in the patients. In practical application, it is necessary to determine whether a tissue sample from a patient reaches a predetermined AKR1C3 expression level and thus meets the conditions where the above drugs of the three patents (CN107530556A, CN108290911A, CN108136214A) are administered, which requires that the IHC assay must have a stable staining result. The inventors attempted to use the IHC assay disclosed in the prior art for the detection of AKR1C3 expression levels in various cancer tumor tissues. It was found that the IHC assay disclosed in the prior art does not meet the practical needs, because the existing IHC assay was a specific IHC assay for a certain enzyme or protein developed by a certain hospital or research institute laboratory targeting a certain tissue for a certain enzyme or protein, instead of the IHC staining assay that can be used in a large-scale commercial kit. These methods did not have good sensitivity, precision and consistency (different laboratories, different operators, different operating times) and were not applied to many different cancer tumor tissues for IHC assay. If the staining results are unstable, the determination of AKR1C3 expression levels will be inaccurate, which in turn will result in an unsatisfactory cancer therapeutic effect.

In order to solve the problem that the IHC assay in the prior art can not be applied to the detection of AKR1C3 expression levels in various cancer tumor tissues and has unstable staining results, the inventors attempted to further improve the existing IHC assay and provided an AKR1C3 detection method, wherein the AKR1C3 detection method can be applied to the detection of AKR1C3 expression levels in various cancer tumor tissues and is stable in staining results, and has good sensitivity, precision and consistency.

One aspect of the present invention provides an AKR1C3 detection method, wherein the AKR1C3 expression levels in isolated formalin-fixed paraffin-embedded (FFPE) human tissue specimen is detected by using immunohistochemical staining method, comprising the following steps:
a) antigen retrieval
   performing antigen retrieval by heating the formalin-fixed paraffin-embedded human tissue specimen at 90~115°C for 17-30 min in the presence of an antigen retrieval solution;
b) primary antibody incubation
   mixing the antigen-retrieved formalin-fixed paraffin-embedded human tissue specimen with a 0.5~5.0 µg/ml concentration of AKR1C3 monoclonal antibody solution for incubation for 25-700 min;
c) secondary antibody incubation
   mixing the primary antibody-incubated formalin-fixed paraffin-embedded human tissue specimen with a 0.5~5.0 µg/ml concentration of secondary antibody solution for incubation for 25-700 min.

The determination sensitivity results show that the analytical sensitivity of the IHC assay provided by the present invention for determining AKR1C3 expression levels in various human cancer tissues has acceptable performance characteristics, which shows the expected staining pattern and localization of AKR1C3 in the test samples and has appropriate performance in quality control, which is consistent with the standard limits for analytical sensitivity studies. The determination precision results show that inter-batch precision (inter-day/operation, inter-operator and inter-instrument) and intra-batch precision were 100% consistency, which is in accordance with the standard limit of ≥95% based on AKR1C3 expression in the cytoplasm and nucleus of tumor cells. The determination consistency results show 100% consistency between pathologists based on AKR1C3 expression in the cytoplasm and nucleus of tumor cells and met the standard limit of ≥90%. Overall, the determination shows acceptable results and demonstrates that it is feasible to use an IHC assay containing the key steps described above for the determination of AKR1C3 expression levels in formalin-fixed paraffin-embedded sample specimen from various human cancer tissues.

In the present invention, the formalin-fixed paraffin-embedded sample specimen is obtained by the following steps: sample preparation, i.e. that tissue samples are treated with formalin and paraffin to obtain formalin-fixed paraffin-embedded (FFPE) samples, and then the FFPE samples are sectiond. The formalin-fixed paraffin-embedded human tissue specimen typically has a thickness of 4 mm.

In the present invention, antigen retrieval is a necessary step in immunohistochemical staining methods before antibody labeling, because the fixation process for tissue usually causes protein cross-linking, which often occurs when formalin fixation is used due to its chemical properties, and an antigen retrieval step is required to re-expose the antigen epitope for antibody binding. The present invention utilizes the action of antigen retrieval solution and heat to re-expose these antigens by placing the specimen to be retrieved in the antigen retrieval solution and then heating it, i.e., by a combined chemical-thermal action to achieve antigen retrieval. A common operation is that the specimen is placed in the antigen retrieval solution, heated in an autoclave together with the container, maintained at a predetermined temperature for a period of time and then removed and cooled down naturally. In a preferred embodiment of the present invention, in the antigen retrieval of step a), the formalin-fixed paraffin-embedded human tissue specimen is heated at 92-102 °C for 18-25 min; more preferably, the formalin fixed paraffin-embedded human tissue specimen is heated at 97 °C for 20 min, which can achieve the best effect: good staining effect on various cancer tumor tissue specimen. The present invention does not limit the cooling temperature after antigen retrieval, which can be naturally cooled to room temperature, or cooled to a certain temperature (e.g., 65 °C) before subsequent operations.

In addition, the pH of the antigen retrieval solution will affect the antigen retrieval effect, and combined with the subsequent use of hematoxylin staining, in a preferred embodiment of the present invention, the antigen retrieval solution has a pH of 2.0 - 9.0; more preferably, the antigen retrieval solution has a pH of 6.0 ~ 9.0; even more preferably, the antigen retrieval solution has a pH of 6.0. The present invention does not limit the composition of the antigen retrieval solution, as long as the pH of the antigen retrieval solution meets the requirements, it can be used in the present invention. The antigen retrieval solution includes, but is not limited to: sodium citrate antigen retrieval solution (Citrate Antigen Retrieval Solution), EDTA antigen retrieval solution (EDTA Antigen Retrieval solution) and the like.

The concentration of AKR1C3 monoclonal antibody solution has a greater impact on the operation of the primary antibody incubation of step b), and it was experimentally determined that 1.0 to 3.0 µg/ml concentration of AKR1C3 monoclonal antibody solution displays good effect, more preferably 1.2 ug/ml concentration of AKR1C3 monoclonal antibody solution, at that time the dilution ratio of AKR1C3 monoclonal antibody solution is 1:2000.

To achieve better results, the AKR1C3 monoclonal antibody solution is obtained by diluting a dilution solution, wherein the dilution solution comprises the following components:
0.02~0.08mol/L of Tris-HCl buffer (tromethamine-hydrochloric acid buffer), containing 0.05~0.15% mass concentration of polyethylene glycol or Tween, and 0.010~0.020 mol/L of sodium azide;

More preferably, the antibody dilution buffer comprises the following components:
0.05 mol/L of Tris-HCl buffer, containing 0.1% mass concentration of polyethylene glycol or Tween, and 0.015 mol/L of sodium azide.

The secondary antibody in immunohistochemical staining method must be an antigen of the anti-primary antibody species, for example: the primary antibody used to detect the B protein of animal A is the animal C anti-B protein of animal A antibody, the secondary antibody should be the animal D anti-animal C antibody. The present invention is to detect human AKR1C3 (protein), the primary antibody used is a mouse anti-human AKR1C3 monoclonal antibody (i.e., mouse AKR1C3 monoclonal antibody), then the secondary antibody is other animals (such as goats, rabbits, horses, donkeys) anti-mouse antibody.

In a preferred embodiment of the present invention, after the secondary antibody incubation of step c), further comprising:
d) staining and sealing
staining the formalin-fixed paraffin-embedded human tissue specimen using hematoxylin, and performing the dehydration and sealing of specimen after staining for subsequent observation and scoring.

In a preferred embodiment of the present invention, before the antigen retrieval of step a), further comprising:
a1) dewaxing and rehydration
dewaxing the formalin-fixed paraffin-embedded human tissue specimen using organic solvent, washing the dewaxed specimen sequentially using alcohols containing different water contents, and finally washing with water; the dewaxing and rehydration operations are performed to replenish the dried sample for various subsequent operations.

As for formalin-fixed paraffin-embedded human tissue specimen, since the embedding with paraffin is performed and the presence of paraffin in the subsequent staining process can cause serious effects, the paraffin must be eluted cleanly using the corresponding organic solvents. The common organic solvents used to elute paraffin without losing specimen include acetone, xylene, toluene, etc., but xylene is more effective and less toxic. After washing the paraffin, the residual organic solvent needs to be washed with an alcohol (methanol or ethanol) dissolved in water, usually ethanol is used. To achieve a better washing effect, a gradient elution method is used, i.e., the dewaxed specimen is first washed with anhydrous ethanol, then washed with ethanol having a volume fraction of 90-97% (e.g. 95%), and finally washed with water.

In a preferred embodiment of the present invention, between the antigen retrieval of step a) and the primary antibody incubation of step b), further comprising:
b1) blocking non-specific antigen
co-incubating the antigen-retrieved formalin-fixed paraffin-embedded human tissue specimen with a blocking solution to block a non-specific antigen;

The blocking of endogenous enzymes and antibodies in the tissue is important for minimizing background staining and reducing false positive staining. This is usually achieved by incubating the sample with a specific buffer that can block non-specific sites to which primary or secondary antibodies may bind. The reagents used in the operation of blocking non-specific antigens are relatively numerous, whose main purpose is to make other proteins, biotin, endogenous enzymes in the sample and other substances (interfering substances) in the IHC detection system do not interfere with the detection results of the AKR1C3 detected in the IHC assay. The use of mouse serum matched with mouse monoclonal antibodies in the present invention can mask all the complex interfering substances and is easy to operate.

In a preferred embodiment of the present invention, an AKR1C3 detection method, wherein the AKR1C3 expression levels in isolated formalin-fixed paraffin-embedded human tissue specimen (FFPE) is detected by using immunohistochemical staining method, comprising the following steps:
a1) dewaxing and rehydration
dewaxing the formalin-fixed paraffin-embedded human tissue specimen with a certain thickness using organic solvent, washing the dewaxed specimen sequentially using alcohols containing different water contents, and finally washing with water;
a) antigen retrieval
performing antigen retrieval by heating the formalin-fixed paraffin-embedded human tissue specimen after dewaxing and rehydration operations at 90~115°C for 17-30 min in the presence of an antigen retrieval solution;
b1) blocking non-specific antigen
co-incubating the antigen-retrieved formalin-fixed paraffin-embedded human tissue specimen with a blocking solution to block a non-specific antigen;
b) primary antibody incubation
mixing the non-specific antigen blocked formalin-fixed paraffin-embedded human tissue specimen with a 0.5~5.0 µg/ml concentration of AKR1C3 monoclonal antibody solution for incubation for 25-700 min;
c) secondary antibody incubation
mixing the primary antibody-incubated formalin-fixed paraffin-embedded human tissue specimen with a 0.5~5.0 µg/ml concentration of secondary antibody solution for incubation for 25-700 min;
d) staining and sealing
staining the formalin-fixed paraffin-embedded human tissue specimen using hematoxylin, and performing the dehydration and sealing of specimen after staining;
e) observation and scoring
observing the stained human tissue specimen, and evaluating the AKR1C3 expression levels in human tissue specimen according to the observed degree of staining.

Certainly, before the operation of the secondary antibody incubation of step c), a further step of blocking non-specific antigen can be introduced, using the serum of the secondary antibody animals as the blocking solution.

The above conditions were used to perform IHC assays on formalin-fixed paraffin-embedded human tissue specimens from various cancers (tumors) including breast cancer, colorectal cancer, esophageal cancer, gastric cancer, hepatocellular carcinoma, non-small cell lung cancer, prostate cancer, renal cell carcinoma, peripheral T-cell lymphoma, and nodular NK/T-cell lymphoma, all of which achieved good staining results, thus demonstrating that the IHC assay for AKR1C3 provided above can be applied to various cancer tumor tissues.

Based on the same inventive concept, another aspect of the present invention provides a diagnostic kit for detecting AKR1C3, comprising:
antigen retrieval solution;
a 0.5~5.0µg/ml concentration of AKR1C3 monoclonal antibody solution;
a 0.5~5.0µg/ml concentration of secondary antibody solution.

In a preferred embodiment of the present invention, the above diagnostic kit for detecting AKR1C3 further comprises:
blocking solution, preferably the blocking solution is a serum of an animal from which the AKR1C3 monoclonal antibody is derived; more preferably, the blocking solution is mouse serum.

In a preferred embodiment of the present invention, the diagnostic kit for detecting AKR1C3 further comprises:
negative control reagent solution; and
instructions documenting the relevant operation methods.

The negative control reagent solution is added to better guarantee the results of the assay, but is not necessary and can be added or not: the presence of the negative control reagent in the results makes it easier to control to check whether the staining assay operation is normal. Preferably, the negative control reagent (NCR) is a commercially available product: FLEX Negative Control, Mouse, (Link) from DAKO Corporation. "Link" means that the reagent is used in combination with the Dako Autostainer Link 48 full automated immunohistochemical staining system used in the following examples.

Based on the same inventive concept, another aspect of the present invention provides use of the above diagnostic kit for detecting AKR1C3 in the preparation of drugs for the treatment of cancer, tumor or cell proliferative disease.

In a preferred embodiment of the present invention, the use described above comprises the following steps:
obtaining AKR1C3 expression levels in isolated formalin-fixed paraffin-embedded human tissue specimen from a patient using the diagnostic kit for detecting AKR1C3 as described above;
administering AKR1C3-activated anticancer drug to the patient whose AKR1C3 expression levels are greater than or equal to predetermined expression levels.

In the present invention, the AKR1C3 expression levels are different for different cancers or tumors, and thus the AKR1C3 expression levels in cancer or tumor tissue specimen suitable for administering drugs with AKR1C3-activated anticancer prodrugs are correspondingly different: high-expression is required for certain cancers, and moderate-expression is sufficient for administering drugs for certain cancers. The AKR1C3 predetermined expression level can be represented by H score and the AKR1C3 predetermined expression levels corresponding to each cancer type can be obtained by statistical method.

In the present invention, AKR1C3-activated anticancer drugs certainly include AKR1C3-activated anticancer prodrugs, i.e., the compounds in the form of prodrugs are reduced under the catalysis of AKR1C3 in the biochemical environment in the cells to finally obtain cytotoxic toxins, thereby exerting toxic effect on cancer cells.

Broadly speaking, an AKR1C3-activated anticancer drug meets, but is not limited to, at least one of the following conditions:
A. in the presence of an AKR1C3 inhibitor (such as TH-3021 disclosed in the three patents above, or compound 36, i.e., 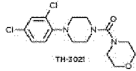 in Flanagan et al., Bioorganic and Medicinal Chemistry (2014), pp. 962-977), the inhibition effect detected of a compound on the proliferation of cancer cells is less than that of cancer cells in the absence of an AKR1C3 inhibitor (such as TH-3021 disclosed in the above three patents); and when the inhibition effect on cancer cell proliferation is quantified using the IC₅₀, then if the IC₅₀ detected of a compound on a certain cancer cell line in the presence of an AKR1C3 inhibitor is greater than that in the absence of an AKR1C3 inhibitor, then the compound can be determined to be an AKR1C3-activated anticancer drug (Lysis-Prodrug). Specifically, Lysis-Prodrug is recited in the following patent documents:
   PCT/US2016/021581, Publication No. WO2016145092A1, corresponding to China Application No. 2016800150788, Publication No. CN107530556A;
   PCT/US2016/062114, Publication No. WO2017087428, corresponding to China Application No. 2016800446081, Publication No. CN108290911A;
   PCT/US2016/025665, Publication No. WO2016161342, corresponding to China Application No. 2016800200132, Publication No. CN108136214A; and
   PCT/NZ2019/050030, Publication No. WO2019190331, as for the compounds disclosed in China Application No. CN2019800234236, Publication No. CN111918864A, and the above patent documents are hereby incorporated into the present patent application text in their entirety.
   wherein the compound disclosed in patent PCT/US2016/021581, Publication No. WO2016145092A1, corresponding to China Application No. 2016800150788, Publication No. CN107530556A; PCT/US2016/062114, Publication No. WO2017087428, corresponding to China Application No. 2016800446081,
   Publication No. CN108290911A; PCT/US2016/025665, Publication No. WO2016161342, corresponding to China Application No. 2016800200132,
   Publication No. CN108136214A, is a Lysis-Prodrug, which is metabolized by final lysis to produce an active parent drug of 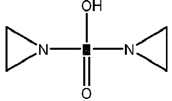 as well as paclitaxel, camptothecin and other drugs; and the compound disclosed in patent PCT/NZ2019/050030, Publication No. WO2019190331, corresponding to China Application No. CN2019800234236, Publication No. CN111918864A, is a Lysis-Prodrug, which is metabolized by final lysis to produce an active parent drug of nitrogen mustard structure drug;
B. the inhibition effect of a compound on the proliferation of cancer cells with different expression levels of AKR1C3 enzyme is significantly different, and the inhibition effect on the proliferation of cancer cells with high AKR1C3 enzyme expression is much greater than that of cancer cells with low AKR1C3 enzyme expression; and when the inhibition effect on the proliferation of cancer cells is quantified using the IC₅₀, then if the IC₅₀ value of a compound on cancer cells with high expression of AKR1C3 enzyme is less than the IC₅₀ value of cancer cells with low expression of AKR1C3 enzyme, then the compound can be determined; specifically as the compounds disclosed in Patent PCT/CN2020/120281, Publication No. WO2021068952A1, which is hereby incorporated into the present patent application text in its entirety.
C. aldo-keto reductase 1C3 (AKR1C3) has the ability to reduce certain aldehyde-ketone compound containing carbon-oxygen double bond to the corresponding alcohol compound containing hydroxyl group. when the inhibition effect of certain aldehyde-ketone compound containing carbon-oxygen double bonds on the proliferation of cancer cells with different expression levels of AKR1C3 enzyme is significantly different, and the inhibition effect on the proliferation of cancer cells with high AKR1C3 enzyme expression is much greater than that of cancer cells with low AKR1C3 enzyme expression, and the difference among the inhibition effects of the corresponding alcohol compound containing hydroxyl groups on the proliferation of cancer cells with different expression levels of AKR1C3 enzyme is small or similar, then the aldehyde-ketone compound containing carbon-oxygen double bonds is AKR1C3-activated anticancer drug (Reduction-Prodrug), and the corresponding alcohol compound containing hydroxyl group is parent drug (Drug); specifically as the compounds disclosed in Patent PCT/IB2020/057285, Publication No. WO2021005586A1, which is hereby incorporated into the present patent application text in its entirety.

The compounds of the general formula, and specific compounds disclosed in the above patent applications are all belong to AKR1C3-activated anticancer drugs/prodrugs, and the above applications are hereby incorporated herein by reference.

Further, compounds of the following structures are preferred, all of which are AKR1C3-activated anticancer drugs/prodrugs: 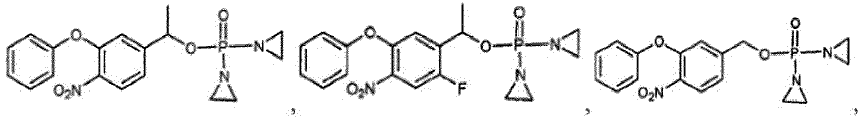 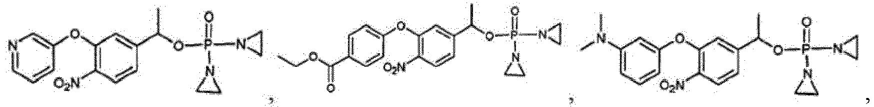 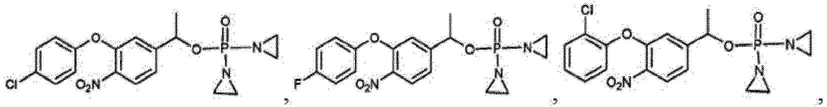 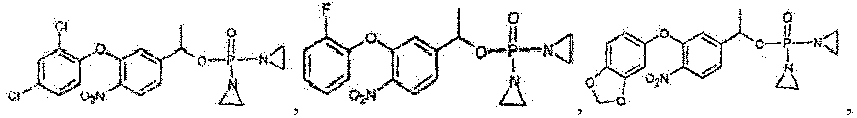 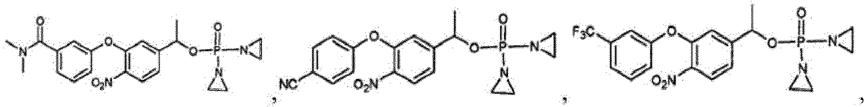 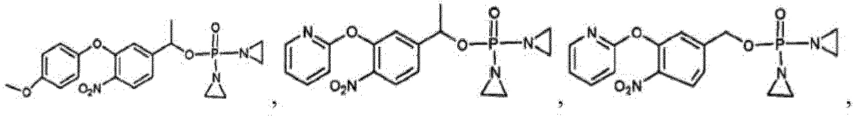 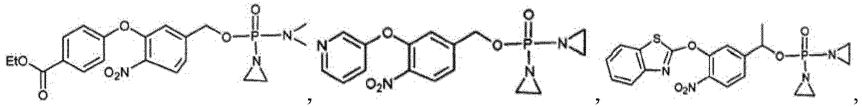 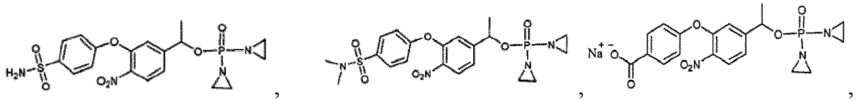 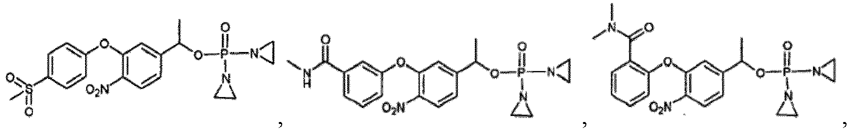 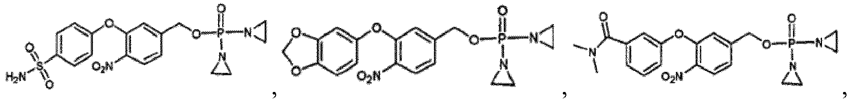 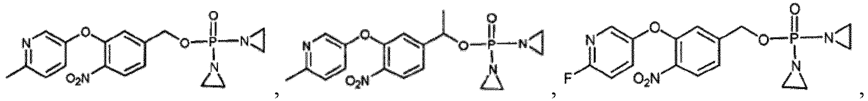 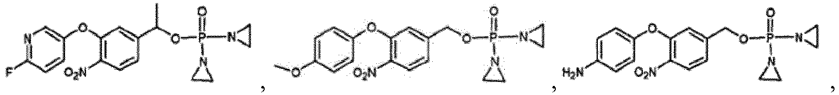 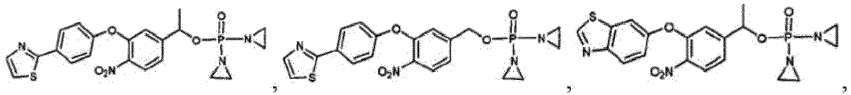 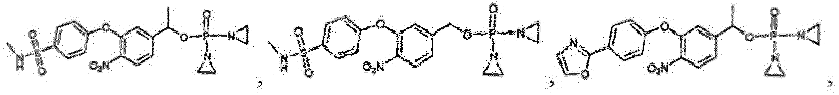 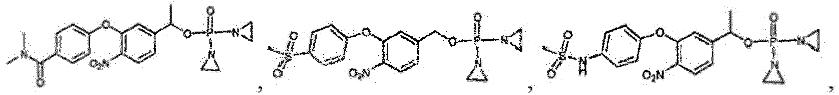 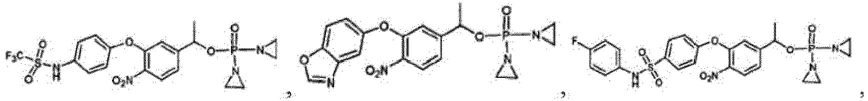 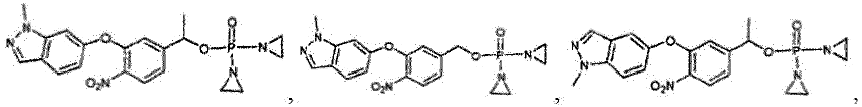 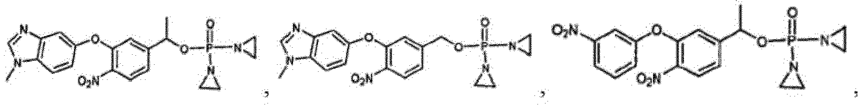 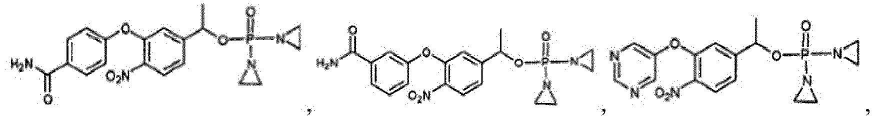 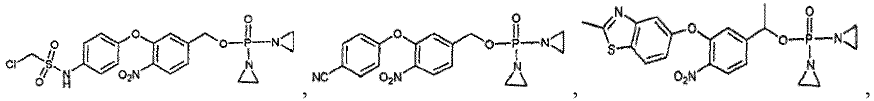 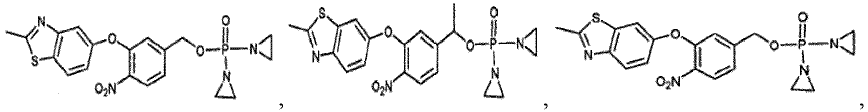 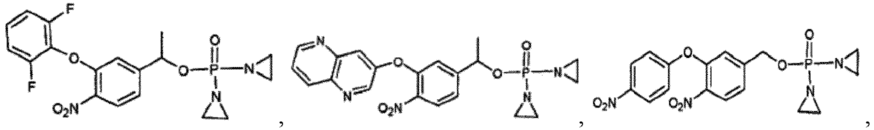 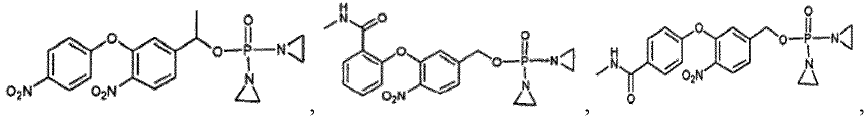 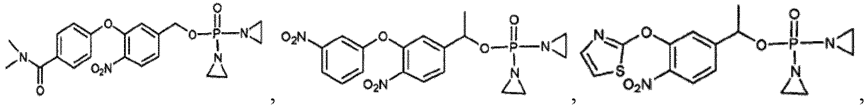 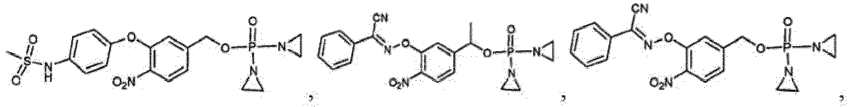 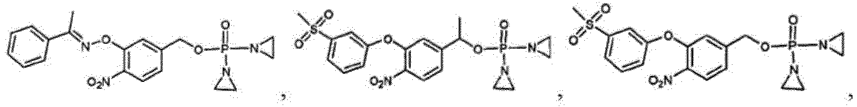 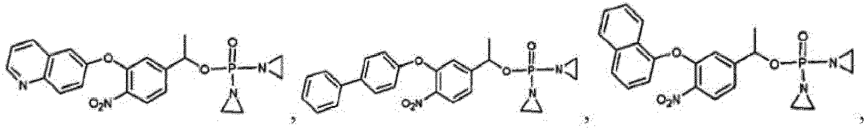 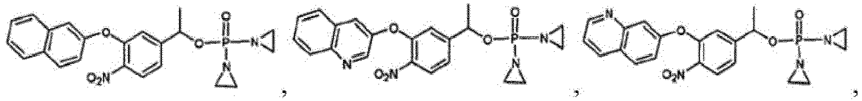 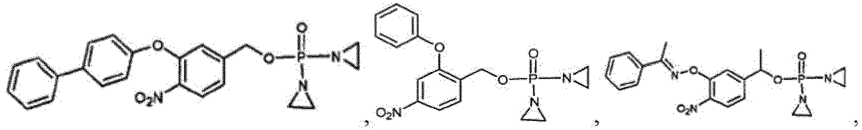 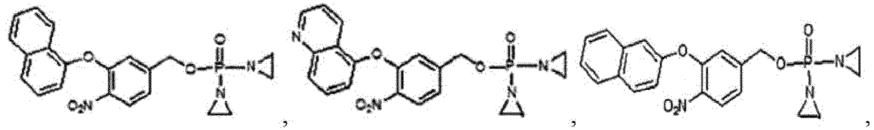 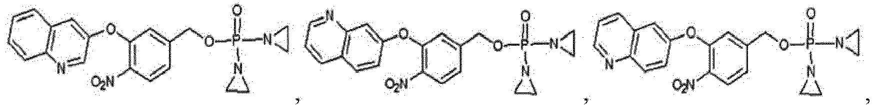 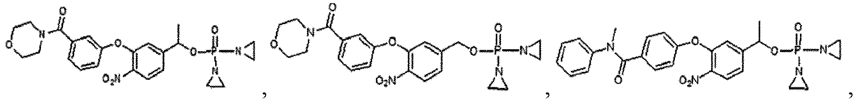 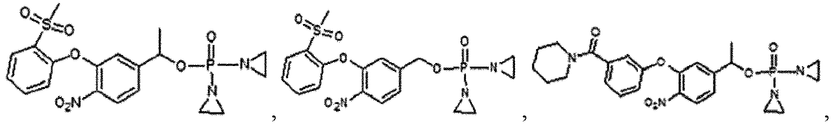 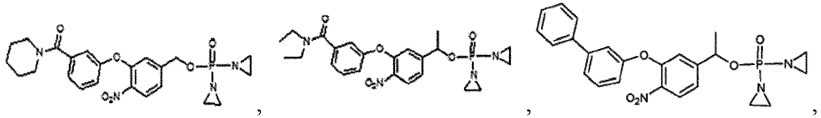 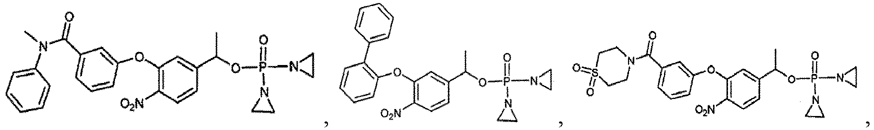 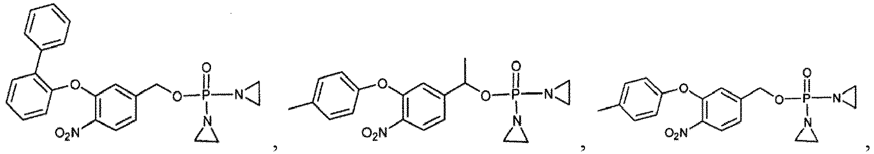 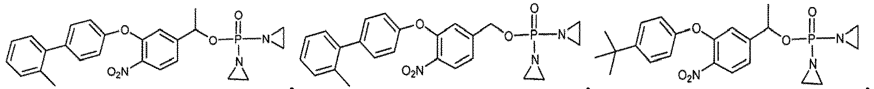 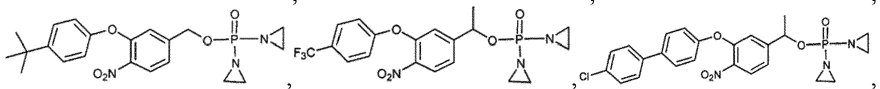 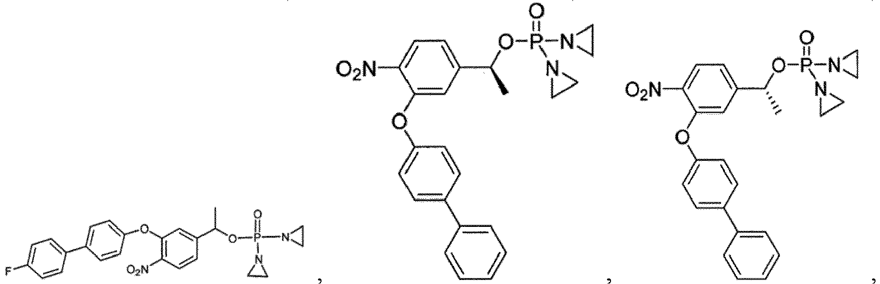 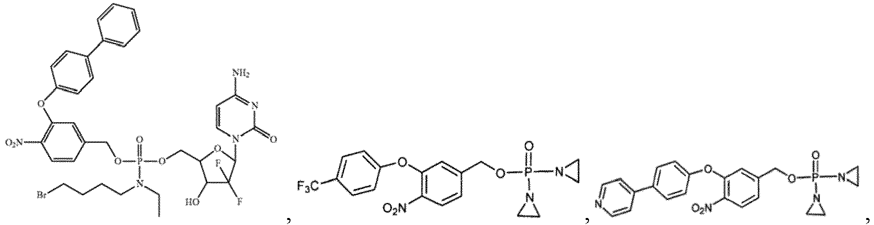 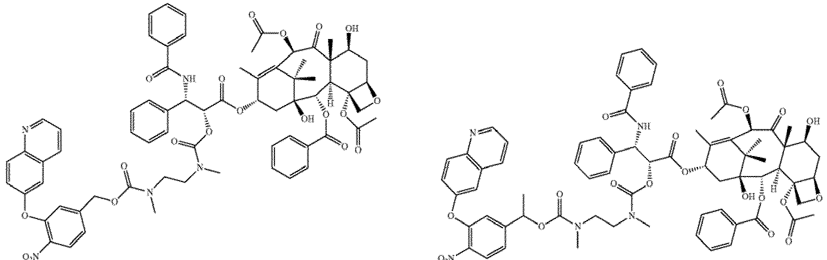 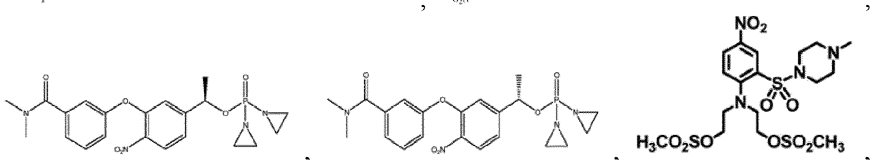 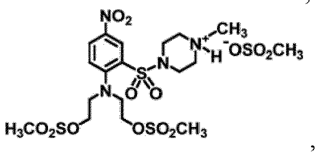 which are AKR1C3-activated anticancer drugs of type A above; or 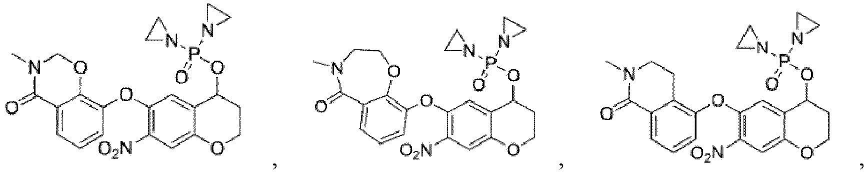 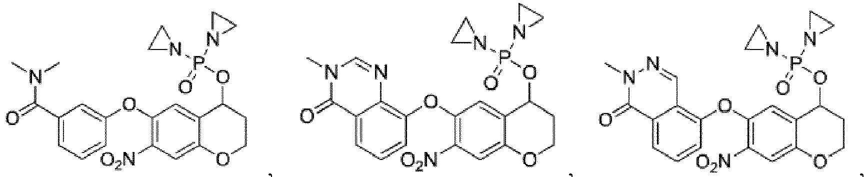 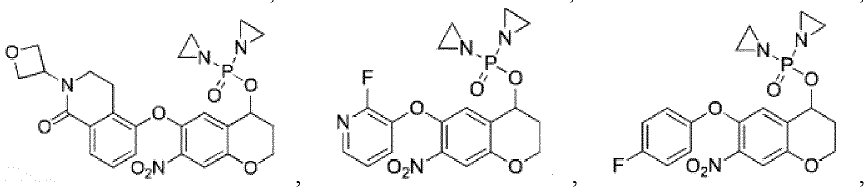 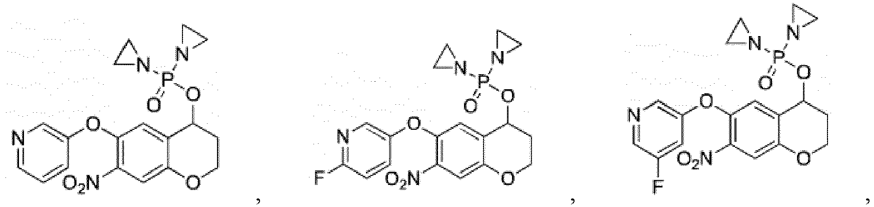 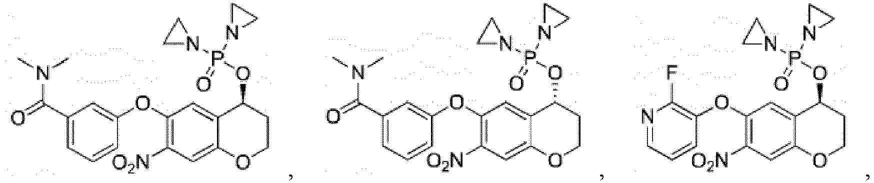 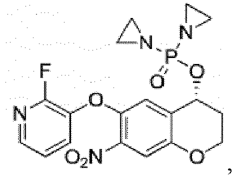 or pharmaceutically acceptable salts or isomers thereof, which are AKR1C3-activated anticancer drugs of type B above; or 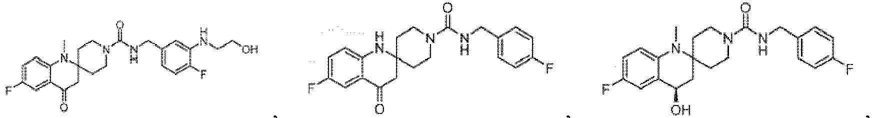 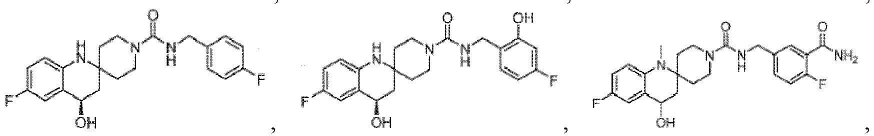 or pharmaceutically acceptable salts or isomers thereof, which are AKR1C3-activated anticancer drugs of type C above.

A total of 46 tissue samples from 9 markers were used in this invention, including 5 RCC (renal cell carcinoma) samples, 5 HCC (hepatocellular carcinoma) samples, 5 NSCLC (non-small cell lung cancer) samples, 5 GC (gastric cancer) samples, 5 PC (prostate cancer) samples, 5 EC (esophageal cancer) samples, 5 CRC (colorectal cancer) samples, 6 peripheral T-cell lymphoma samples and 5 NK/T-cell lymphoma samples.

As for each staining operation, normal colon tissue having both positive and negative components was used as a double positive and negative tissue quality control.

All of the above samples were formalin-fixed paraffin-embedded (FFPE) human tissue specimens and were cut into tissue specimens of 4 µm thickness, placed on positively charged slides and stored at ambient temperature until stained.

The experimental biochemical and chemical reagents are listed in Table 1 below.

**Table 1: Experimental biochemical reagents and chemical reagents**

| Material | Dilution ratio used | Producer and Number | Main components obtained according to product manual |
|---|---|---|---|
| Anti-AKR1C3 antibody, mouse monoclonal (2.4 mg/ml), batch number 127M4838V | Diluted at different concentrations | Sigma A6229 | Anti-AKR1C3 antibody, mouse monoclonal NP6.G6.A6, purified from hybridoma cell culture |
| FLEX negative control, mouse, (Link), batch number 10139529 | Ready-to-use | Dako IR750 | / |
| EnVision^{™} FLEX+(Link) Kit, batch number 20056873 | Ready-to-use | Dako K8002 | / |
| EnVision^{™} FLEX Antigen Retrieval Solution, High pH, pH9.0, batch number 20058957 | 1:50 | Dako K8004 | TRIS, EDTA, Disodium EDTA, Sodium citrate |
| EnVision^{™} FLEX Antigen Retrieval Solution, Low pH, pH 6.0, batch number 10144956 | 1:50 | Dako K8005 | TRIS, EDTA, Disodium EDTA, Sodium citrate |
| EnVision^{™} FLEX Hematoxylin (Link) batch number 20061378 | Ready-to-use | Dako K8008 | / |
| SignalStain Antibody Diluent, batch number 26 | Ready-to-use | Cell Signaling Technologv 8112L | / |
| Antibody Diluent, batch number 10142590 | Ready-to-use | Dako S3022 | Polyethylene glycol, Tween, NaN₃ |
| Ethanol, batch number 8190108 | Ready-to-use | XiLONG SCIENTIFIC 1030001-02-01 | / |
| Xylene, batch number 180808 | Ready-to-use | XiLONG SCIENTIFIC | / |
| Mounting agent, batch number 17081515 | Readv-to-use | Dako CS703 | Toluene, Xylene |
| Negative control, batch number G06157 | Readv-to-use | Ventana 760-2014 | / |
| Antibody Dilution Buffer, batch number Y18235 | Ready-to-use | Ventana ADB250 | / |
| OptiView DAB IHC Assay Kit, batch number E02544 | Ready-to-use | Ventana 760-700 | / |
| Hematoxylin II. batch number E12781 | Readv-to-use | Ventana 790-2208 | / |
| Bluing Reagent, batch number E14723 | Ready-to-use | Ventana 760-2037 | / |
| EZ Prep Concentrate (10x), batch number 331-986-01 | 1:10 | Ventana 950-102 | / |
| Reaction Buffer Concentrate (10X), batch number 345-950-01 | 1:10 | Ventana 950-300 | / |
| ULTRA LCS (pre-dilution), batch number E20921 | Ready-to-use | Ventana 650-210 | / |

### Instruments

Ventana Benchmark Ultra full automated immunohistochemistry stainer (also known as tissue specimen stainer, with the serial numbers: 311434, 316829)
Dako Autostainer Link 48 full automated immunohistochemical staining system (also known as tissue specimen stainer with the serial numbers: AS5085D1611, AS2370D1203)
DAKO PT Link full automated immunohistochemistry pre-processing system (Serial numbers: MY1716P184, PT3543Y1310)
Sakura Tissue-Tek DRS Slide Stainer (Serial number: 49310219-0409)
Sakura Slide Heater (Serial number: 14881799-0409)
Sakura Slicer (Serial number: 1429-1407)
NIKON Microscope (Serial number: 940776)
HANNA pH meter (Serial number: 08678109)
Positively charged microscope slides
Aperio Scanscope XT digital specimen scanning system (Serial number: SS001403)

The technical solutions provided by the present invention are further described below in combination with specific examples. The following examples are only intended to illustrate the present invention and do not limit the protection scope of the present invention.

The standards for evaluating the staining results involved in the following examples were as follows:

### (1) Staining description

Cells labelled by AKR1C3 antibody show cytoplasmic and/or nuclear staining.

### (2) General scoring guidelines

The interpretation of any staining or lack of staining was achieved by morphological studies using appropriate quality controls and evaluated by qualified pathologists (GH and KZ) to determine the localization, distribution and intensity of AKR1C3 staining in the tumour sample.

Reactivity evaluation includes the following aspects:
Cellular localization of the staining
Intensity of staining
Subcellular localization
Percentage of cellular staining in the region of interest

The AKR1C3 determination was evaluated on a semi-quantitative scale and the percentage of cells staining at the following four levels (0, 1+, 2+ and 3+) was recorded for cytoplasmic and nuclear staining.

### (3) Scoring standard for tumour samples

Using the H-Score: % of nucleus-cytoplasm stained tumour cells (total values from 0 to 3+ should not exceed 100) was used to score the degree of staining (i.e., the level of AKR1C3 enzyme expression).
0 (unstained): values between 0 and 100
Tumour cell nucleus-cytoplasm 1+ (weak staining): values between 0 and 100
Tumour cell nucleus-cytoplasm 2+ (moderate staining): values between 0 and 100
Tumour cell nucleus-cytoplasm 3+ (strong staining): values between 0 and 100
Total % of nucleus-cytoplasm positive staining: values between 0 and 100

A total H-score will be calculated based on the proportional tumour score for each intensity. The final H-score was calculated as follows: H-score = (% weak [1+] x 1) + (% moderate [2+] x 2) + (% strong [3+] x 3)

There was no specific cut-off score assigned for clinical interpretation to determine the positive and negative states of AKR1C3 during the present verification, which may be determined after completing the clinical trial.

The total positive % score ± 10% was defined as the consistency of the same sample between pathologists. However, if the same case was scored as 0 and 1% by the pathologists, it should be considered as inconsistency.

### Example 1 Establishment of the AKR1C3 detection method

In this example, the existing methods provided by the commercially available Dako Autostainer Link 48 platform and the Ventana Benchmark Ultra platform were pre-verified firstly. The pre-verification results showed that neither of the existing methods provided by the two platforms was applicable to the IHC method for AKR1C3. Therefore, there is need to optimize the conditions for Ventana Benchmark Ultra and Dako Autostainer Link 48.

Based on the principles of IHC and in combination with the staining results of pre-verification, the inventors determined that the conditions to be optimized included:
Optimization of Ab concentration and incubation time
Optimization of antigen retrieval conditions
Optimizing the conditions of the detection system

### 1.1 Optimization of Ab concentration

The pre-verification results showed that AKR1C3 1:100 and 1:500 had extremely strong background staining. Staining at 1:4000 was too weak. The main attention was then given to AKR1C3 1:1000 and 1:2000 in the next step, although both also showed background staining.

### 1.2. Optimization of antigen retrieval conditions

Staining tests were carried out using high pH antigen retrieval solutions (pH 9.0) and the results were shown in Table 2:

**Table 2 Results of staining tests using antigen retrieval solution at high pH**

| Sample for testing | Antigen retrieval solution and retrieval conditions | Antibody dilution buffer | AKR1C3 dilution ratio | Incubation time for primary antibody (containing negative control reagent and AKR1C3 solution) | Incubation time for secondary antibody | Representative figure |
|---|---|---|---|---|---|---|
| 1 CRC Sample: *335933-P* | High pH, preheated at 97°C for 20 min and cooled to 65°C | Dako dilution buffer S3022 | 1:1000 | 20 min | 20 min | Figure 1a |
| | | Dako dilution buffer S3022 | 1:2000 | 45 min | 30 min | Figure 1b |
| | | Dako dilution buffer S3022 | 1:2000 | 60 min | 30 min | Figure 1c |

Staining tests were carried out using low pH antigen retrieval solutions (pH 6.0) and the results were shown in Table 3:

**Table 3 Results of staining tests using low pH antigen retrieval solution**

| Sample for testing | Antigen retrieval solution and retrieval conditions | Antibody dilution buffer | AKR1C3 dilution ratio | Incubation time for primary antibody (containing negative control reagent and AKR1C3 solution) | Incubation time for secondary antibody | Representative figure |
|---|---|---|---|---|---|---|
| 1 CRC Sample: *335933-P* | Low pH, preheated at 97°C for 20 min and cooled to 65°C | Dako dilution buffer S3022 | 1:1000 | 30 min | 30 min | Figure 2a |
| | | | 1:2000 | 45 min | 30 min | Figure 2b |
| | | | 1:2000 | 60 min | 30 min | Figure 2c |

High pH antigen retrieval solutions had extremely strong background staining. However, if the pH was low, the background staining was significantly reduced. Low pH antigen retrieval solution will be used for the present verification (Scanscope scan 1x).

### 1.3 Optimal testing in further samples

Optimal testing was carried out in further samples including normal tissues and various solid tumours, using low pH antigen retrieval solution to determine the optimal Ab concentration and incubation time.

**Table 4 Results of IHC staining of gastric cancer samples at low pH**

| Sample for testing | Antigen retrieval solution and retrieval conditions | Antibody dilution buffer | AKR1C3 dilution ratio | Incubation time for primary antibody (containing negative control reagent and AKR1C3 solution) | Incubation time for secondary antibody | Representative figure |
|---|---|---|---|---|---|---|
| gastric cancer: F180723A5 F180684A3 | Low pH, preheated at 97°C for 20 min and cooled to 65°C | Dako dilution buffer S3022 | 1:1000 | 30 min | 30 min | Figure 3a |
| | | | 1:2000 | 45 min | 30 min | Figure 3b |
| | | | 1:2000 | 45 min | 45 min | Figure 3c |

As can be seen from the staining results in Figures 3a-3c, the background/non-specific staining appeared in Figure 3a, the appropriate staining was in Figure 3b and the background/non-specific staining appeared in Figure 3c.

**Table 5 Results of IHC staining of breast cancer samples at low pH**

| Sample for testing | Antigen retrieval solution and retrieval conditions | Antibody dilution buffer | AKR1C3 dilution ratio | Incubation time for primary antibody (containing negative control reagent and AKR1C3 solution) | Incubation time for secondary antibody | Representative figure |
|---|---|---|---|---|---|---|
| breast cancer: F162870A5 F130368B3 | Low pH, preheated at 97°C for 20 min and cooled to 65°C | Dako dilution buffer S3022 | 1:1000 | 30 min | 30 min | Figure 4a |
| | | | 1:2000 | 45 min | 30 min | Figure 4b |
| | | | 1:2000 | 45 min | 45min | Figure 4c |

As can be seen from the staining results in Figures 4a-4c, the background/non-specific staining appeared in Figure 4a, the appropriate staining was in Figure 4b and the background/non-specific staining appeared in Figure 4c.

It can be determined that the optimal staining performance was shown under the following conditions: low pH antigen retrieval solution, 97°C for 20 min; AKR1C3 diluted 1:2000, incubation time: 45 min; HRP (horseradish peroxidase) incubation time: 30 min, as determined by staining of positive and negative tissue components and specific positive staining regarding cellular localization and staining intensity range, yielding the optimal signal-to-noise ratio. This optimal staining protocol will then be used for the verification (Scanscope scan 1x).

### 1.4 QC (Quality Control) verification

Five normal colon tissues were pre-verified using the optimal staining protocol described above to test the staining performance and verify whether it was an appropriate QC quality control for the study.

**Table 6 Staining results of normal colon tissue under the optimal staining protocol**

| Sample for testing | Antigen retrieval solution and retrieval conditions | Antibody dilution buffer | AKR1C3 dilution ratio | Incubation time for primary antibody (containing negative control reagent and AKR1C3 solution) | Incubation time for secondary antibody | Representative figure |
|---|---|---|---|---|---|---|
| normal colon tissue: 391761-YN | Low pH, preheated at 97°C for 20 min and cooled to 65°C | Dako dilution buffer S3022 | 1:1000 | 30 min | 30 min | Figure 5a |
| | | | 1:2000 | 45 min | 30 min | Figure 5b |
| | | | 1:2000 | 30 min | 30 min | Figure 5c |

As can be seen from the staining results in Figures 5a-5c, the background/non-specific staining appeared in Figure 5a, the appropriate staining was in Figure 5b and the weakly specific staining appeared in Figure 5c. Using the optimal staining protocol, all five normal colon tissues showed consistent and optimal staining performance (low pH antigen retrieval solution, 97°C for 20 min; AKR1C3 diluted 1:2000, incubation time: 45 min; HRP incubation time: 30 min), as determined by staining of positive and negative tissue components and specific positive staining regarding cellular localization and staining intensity range, yielding the optimal signal-to-noise ratio, as shown in FIG. 6. Normal colon tissue with both positive and negative components was then used as a double positive and negative tissue quality control and used for each staining operation of the study.

The optimal staining conditions were determined based on the results of these tests and were shown in Table 7.

**Table 7 Optimal staining conditions**

| | |
|---|---|
| Low pH TRS | 97°C 20 min |
| AKR1C3 dilution | 1 :2000 (1.2 ug/ml) |
| Incubation time for primary antibody | 45 min |
| Incubation time for secondary antibody/HRP | 30 min |

That is, the finalized AKR1C3 detection method includes the following steps:
an antigen retrieval step
performing antigen retrieval by heating the formalin-fixed paraffin-embedded human tissue specimen at 97°C for 20 min in the presence of an antigen retrieval solution of pH 6.0;
a primary antibody incubation step
mixing the formalin-fixed paraffin-embedded human tissue specimen with a 1.2 µg/ml concentration of AKR1C3 monoclonal antibody solution and a negative control reagent solution for incubation for 45 min; wherein the negative control reagent solution was a commercially available product: FLEX Negative Control, Mouse, (Link) from DAKO Corporation;
a secondary antibody incubation
mixing the formalin-fixed paraffin-embedded human tissue specimen with a 1.2 µg/ml concentration of secondary antibody solution for incubation for 30 min.

IHC staining was performed using optimal staining conditions for normal tonsil tissue, normal gastric tissue, normal colon tissue, non-small cell lung cancer, gastric cancer, breast cancer, hepatocellular carcinoma and colorectal cancer, as shown in FIGs. 7-12. The staining of various normal tissues and solid tumours showed the optimal signal-to-noise ratio, which was determined by staining of positive and negative tissue components and specific positive staining regarding cellular localization and staining intensity range. Normal tissues: stromal cells and endothelial cells may show different intensity levels of staining.

### 1.5 QC determination

Normal colon tissue with both positive and negative components will be used as a double positive and negative tissue quality control for each staining operation and will be used for the following verification operations and future *in vivo* studies.

As shown in FIG. 13, staining tests for normal colon tissue with positive and negative components were performed using the optimal protocol described above, and photographs were scanned at different magnification times at the same time. It is clearly found that it was obvious for the distinction between positive and negative stainingand the staining effect was good, so that it was convenient for subsequent scoring by pathologists to determine the level of AKR1C3 expression.

### Example 2 Sensitivity of analytical methods

### 2.1 Test method

A total of 46 tissue samples, including 5 RCC, 5 HCC, 5 NSCLC, 5 GC, 5 PC, 5 EC, 5 CRC, 6 peripheral T-cell lymphoma tissue samples and 5 NK/T-cell lymphoma tissue samples, were stained with AKR1C3 antibody to evaluate the sensitivity of this IHC assay.

Normal colon tissue used as a double positive and negative tissue quality control and a negative control reagent (NRC) for each sample were included in each staining operation, which were first evaluated by QBEJ pathologists and must show the expected acceptable staining for the biomarker.

Standard limits:
(1) Samples stained with NRC must show 0 specific staining and <1+ intensity of background staining.
(2) Samples stained with AKR1C3 antibody must show <1+ of non-specific background staining intensity.
(3) Cells stained with AKR1C3 antibody must show appropriate cellular localization.

### 2.2 Test results

The results of the sensitivity evaluation of the 46 tissue samples were summarized in Table 8.

All results of the quality control, including positive and negative quality control for each operation and negative control reagents for each sample, were evaluated first and showed the expected acceptable staining for the biomarker.

**Table 8 Summary results of the analytical sensitivity studies**

| Sample No. | Sample ID | Marker | Different staining intensity of nucleus-cytoplasm of tumour cells % | | | | Total positive nucleus-cytoplasm stained% | H Score | Note |
|---|---|---|---|---|---|---|---|---|---|
| | | | 0 | 1+ | 2+ | 3+ | | | |
| 1 | F150984A1 | HCC | 0 | 5 | 0 | 95 | 100 | 290 | NA |
| 2 | F150985A16 | HCC | 1 | 2 | 2 | 95 | 99 | 291 | NA |
| 3 | F151093A4 | HCC | 5 | 15 | 40 | 40 | 95 | 215 | NA |
| 4 | F151286A5 | HCC | 0 | 0 | 0 | 100 | 100 | 300 | NA |
| 5 | F151288A3 | HCC | 79 | 20 | 0 | 1 | 21 | 23 | NA |
| 6 | F151053A4 | NSCLC | 100 | 0 | 0 | 0 | 0 | 0 | < 1% tumour cells stained |
| 7 | F151419A5 | NSCLC | 40 | 50 | 10 | 0 | 60 | 70 | NA |
| 8 | F151588A4 | NSCLC | 100 | 0 | 0 | 0 | 0 | 0 | < 1% tumour cells stained |
| 9 | F152115A1 | NSCLC | 43 | 0 | 55 | 2 | 57 | 116 | NA |
| 10 | F152186A6 | NSCLC | 80 | 0 | 15 | 5 | 20 | 45 | NA |
| 11 | F161764A4 | PC | 100 | 0 | 0 | 0 | 0 | 0 | NA |
| 12 | F163331A7 | PC | 100 | 0 | 0 | 0 | 0 | 0 | NA |
| 13 | F183408A5 | PC | 100 | 0 | 0 | 0 | 0 | 0 | NA |
| 14 | F183409A3 | PC | 100 | 0 | 0 | 0 | 0 | 0 | NA |
| 15 | F183410A4 | PC | 100 | 0 | 0 | 0 | 0 | 0 | NA |
| 16 | F152469A1 | RCC | 0 | 5 | 90 | 5 | 100 | 200 | NA |
| 17 | F153482A6 | RCC | 5 | 0 | 10 | 85 | 95 | 275 | NA |
| 18 | F160249A3 | RCC | 10 | 30 | 60 | 0 | 90 | 150 | NA |
| 19 | F160840A4 | RCC | 10 | 50 | 0 | 40 | 90 | 170 | NA |
| 20 | F161270A10 | RCC | 5 | 30 | 50 | 15 | 95 | 175 | NA |
| 21 | F152459A4 | GC | 85 | 5 | 0 | 10 | 15 | 35 | NA |
| 22 | F152481A3 | GC | 0 | 10 | 20 | 70 | 100 | 260 | NA |
| 23 | F153051A6 | GC | 0 | 0 | 0 | 100 | 100 | 300 | NA |
| 24 | F153077A8 | GC | 60 | 0 | 20 | 20 | 40 | 100 | NA |
| 25 | F153286A4 | GC | 0 | 0 | 80 | 20 | 100 | 220 | NA |
| 26 | F150837A1 | EC | 5 | 0 | 0 | 95 | 95 | 285 | NA |
| 27 | F151725A1 | EC | 0 | 60 | 20 | 20 | 100 | 160 | NA |
| 28 | F152367A7 | EC | 0 | 0 | 5 | 95 | 100 | 295 | NA |
| 29 | F160053A13 | EC | 30 | 0 | 15 | 55 | 70 | 195 | NA |
| 30 | F160743A7 | EC | 2 | 0 | 3 | 95 | 98 | 291 | NA |
| 31 | F150576A4 | CRC | 0 | 0 | 0 | 100 | 100 | 300 | NA |
| 32 | F151182A1 | CRC | 5 | 15 | 60 | 20 | 95 | 195 | NA |
| 33 | F151461A9 | CRC | 85 | 15 | 0 | 0 | 15 | 15 | NA |
| 34 | F151653A1 | CRC | 30 | 30 | 30 | 10 | 70 | 120 | NA |
| 35 | F152160A5 | CRC | 5 | 0 | 95 | 0 | 95 | 190 | NA |
| 36 | F083987A3 | PTCL | 97 | 0 | 3 | 0 | 3 | 6 | NA |
| 37 | F084245A8 | PTCL | 100 | 0 | 0 | 0 | 0 | 0 | NA |
| 38 | F102289A8 | PTCL | 100 | 0 | 0 | 0 | 0 | 0 | NA |
| 39 | F162428A6 | PTCL | 30 | 0 | 65 | 5 | 70 | 145 | NA |
| 40 | F170679A13 | PTCL | 100 | 0 | 0 | 0 | 0 | 0 | NA |
| 41 | F172071A4 | PTCL | 95 | 5 | 0 | 0 | 5 | 5 | NA |
| 42 | F084112A3 | NK/T | 100 | 0 | 0 | 0 | 0 | 0 | NA |
| 43 | F093801A3 | NK/T | 100 | 0 | 0 | 0 | 0 | 0 | NA |
| 44 | F171215A1 | NK/T | 100 | 0 | 0 | 0 | 0 | 0 | NA |
| 45 | F182108A1 | NK/T | 100 | 0 | 0 | 0 | 0 | 0 | NA |
| 46 | F185255A8 | NK/T | 100 | 0 | 0 | 0 | 0 | 0 | NA |

The results of the analytical sensitivity operation for all quality control slides and tumour samples demonstrated that the AKR1C3 antibody showed the expected staining and localization of AKR1C3 in normal colon tissue quality control and tumour samples, in accordance with the standard limits mentioned in Example 3.

### Example 3 Precision of the analytical method

### 3.1 Test methods

Using the five samples with different AKR1C3 expression from the analytical sensitivity study described above, three staining operations were performed by two operators on two instruments for three non-consecutive days to evaluate inter-batch (inter-day/operation, inter-operator and inter-instrument) precision and intra-batch precision. The design of the precision study was shown in Table 9 below.

The inter-batch precision included:

### (1) inter-day/inter-batch precision, requiring that:

Staining was performed in a non-consecutive 3-day cycle.

### 1 NRC section and 1 AKR1C3 specimen per sample

### (2) inter-operator and inter-instrument precision, requiring that:

Two staining platforms were utilized during the 3 staining operations and involved two operators.

### (3) intra-batch precision

One operation involved 4 serial specimens for intra-batch precision.

### 1 NRC section and 3 AKR1C3 specimens per sample

**Table 9 Experimental design for precision studies**

| inter-batch 3 operation, 5 samples | | | Intra-batch 1 operation, 5 samples |
|---|---|---|---|
| Inter-operator | Inter-instrument | Inter-bach / inter-day 3 different operations / 3 non-consecutive days | Intra-batch |
| Operator 1 | Instrument 1 | | 1 NRC specimen and 3 AKR1C3 specimens per sample |
| Operator 2 | Instrument 2 | | |
| Same instrument | Same operator | | |
| 1 NRC specimen and 1 AKR1C3 specimen per sample | 1 NRC specimen and 1 AKR1C3 specimen per sample | | |
| | | 1NRC specimen and 1 AKR1C3 specimen per sample | |
| Operation 1: | Operation 1: | Operation 1: | Operation 3: |
| Instrument 1 | Instrument 1 | Instrument 1 | Instrument 2 |
| Operator 1 | Operator 1 | Operator 1 | Operator 1 |
| Day A | Day A | Day A | Day C |
| Operation 2: | Operation 3: | Operation 2: | ---- |
| Instrument 1 | Instrument 2 | Instrument 1 | |
| Operator 2 | Operator 1 | Operator 2 | |
| Day B | Day C | Day B | |
| ----- | ----- | Operation 3: | ---- |
| | | Instrument 2 | |
| | | Operator 1 | |
| | | Day C | |

Normal colon tissue used as a double positive and negative tissue quality control and a negative control reagent (NRC) for each sample were included in each staining operation, which were first evaluated by pathologists and must show the expected acceptable staining for the biomarker. The interpretation of results and scoring standard were described in detail in the "Scoring standard for tumour samples".

Standard limits:
The standard limit of inter-batch and intra-batch consistency was maintained ≥95% based on AKR1C3 expression in the cytoplasm and nucleus of tumour cells as described in detail in the "Scoring standard for tumour samples".

### 3.2 Test results

Using the five samples with different AKR1C3 expression from the analytical sensitivity study described above, three staining operations were performed by two operators on two instruments for three non-consecutive days to evaluate inter-batch (inter-day/operation, inter-operator and inter-instrument) precision and intra-batch precision. The results were summarized in Table 10.

**Table 10 Summary results of precision studies**

| Sample ID | | Different staining intensity of nucleus-cytoplasm of tumour cells% | | | | Total positive nucleus-cytoplasm stained% | H Score | Consistency Yes/No |
|---|---|---|---|---|---|---|---|---|
| | | | | | | | | (Total positive nucleus-cytoplasm stained% ± 10%) |
| | | 0 | 1+ | 2+ | 3+ | | | |
| F151653A1 | Dav A/Operation 1 | 35 | 25 | 30 | 10 | 65 | 115 | Yes |
| | Day A/Operation 2 | 30 | 30 | 30 | 10 | 70 | 120 | |
| | Day C Operation 3 Repeat 1 | 30 | 30 | 30 | 10 | 70 | 120 | |
| | Day C Operation 3 Repeat 2 | 30 | 30 | 30 | 10 | 70 | 120 | |
| | Day C Operation 3 Repeat 3 | 35 | 25 | 30 | 10 | 65 | 115 | |
| F151725A1 | Dav A/Operation 1 | 0 | 60 | 20 | 20 | 100 | 160 | Yes |
| | Day A/Operation 2 | 10 | 50 | 20 | 20 | 90 | 150 | |
| | Day C Operation 3 Repeat 1 | 0 | 50 | 30 | 20 | 100 | 170 | |
| | Day C Operation 3 Repeat 2 | 0 | 50 | 30 | 20 | 100 | 170 | |
| | Day C Operation 3 Repeat 3 | 0 | 50 | 30 | 20 | 100 | 170 | |
| F183410A4 | Day A/Operation 1 | 100 | 0 | 0 | 0 | 0 | 0 | Yes |
| | Dav A/Operation 2 | 100 | 0 | 0 | 0 | 0 | 0 | |
| | Day C Operation 3 Repeat 1 | 100 | 0 | 0 | 0 | 0 | 0 | |
| | Day C Operation 3 Repeat 2 | 100 | 0 | 0 | 0 | 0 | 0 | |
| | Day C Operation 3 Repeat 3 | 100 | 0 | 0 | 0 | 0 | 0 | |
| F151286A5 | Day A/Operation 1 | 0 | 0 | 0 | 100 | 100 | 300 | Yes |
| | Dav A/Operation 2 | 0 | 0 | 0 | 100 | 100 | 300 | |
| | Day C Operation 3 Repeat 1 | 0 | 0 | 0 | 100 | 100 | 300 | |
| | Day C Operation 3 Repeat 2 | 0 | 0 | 0 | 100 | 100 | 300 | |
| | Day C Operation 3 Repeat 3 | 0 | 0 | 0 | 100 | 100 | 300 | |
| F152459A4 | Dav A/Operation 1 | 83 | 5 | 2 | 10 | 17 | 39 | Yes |
| | Day A/Operation 2 | 82 | 5 | 3 | 10 | 18 | 41 | |
| | Day C Operation 3 Repeat 1 | 82 | 5 | 3 | 10 | 18 | 41 | |
| | Day C Operation 3 Repeat 2 | 83 | 5 | 2 | 10 | 17 | 39 | |
| | Day C Operation 3 Repeat 3 | 83 | 5 | 2 | 10 | 17 | 39 | |

The AKR1C3 IHC assay was performed by two operators performing three staining operations on five samples with different AKR1C3 expression on two instruments for three non-consecutive days and their precision showed replicable results. Based on AKR1C3 expression in the cytoplasm and nucleus of tumour cells as described in detail above, the inter- and intra-operations showed 100% consistency, which was in accordance with the standard limit of ≥95%.

### Example 4 Consistency of analytical methods

### 4.1 Test methods

Two pathologists (GH and KZ) independently evaluated the 46 tissue samples used for the analytical sensitivity study.

The interpretation of results and scoring standard were described in detail in the "Scoring standard for tumour samples".

Standard limits:
The standard limit of consistency between pathologists was ≥90% based on AKR1C3 expression in the cytoplasm and nucleus of tumour cells as described in detail in the "Scoring standard for tumour samples".

### 4.2 Test results

Two pathologists (GH and KZ) independently evaluated the 46 tissue samples used for the analytical sensitivity study. The results were summarized in Table 11.

**Table 11 Result of consistency between pathologists**

| Sample No. | Sample ID | Staining evaluation for AKR1C3 by KZ | | | | | | Staining evaluation for AKR1C3 by GH | | | | | | Consistency Yes/No |
|---|---|---|---|---|---|---|---|---|---|---|---|---|---|---|
| | | Different staining intensity of nucleus-cytoplasm of tumour cells % | | | | Total positive nucleus-cytoplasm stained% | H Score | Different staining intensity of nucleus-cytoplasm of tumour cells % | | | | Total positive nucleus-cytoplasm stained% | H Score | |
| | | | | | | | | | | | | | | (Total positive nucleus-citoplasm stained% ± 10%) |
| | | 0 | 1+ | 2+ | 3+ | | | 0 | 1+ | 2+ | 3+ | | | |
| 1 | F150984A1 | 0 | 5 | 15 | 80 | 100 | 275 | 0 | 5 | 0 | 95 | 100 | 290 | Yes |
| 2 | F150985A16 | 0 | 0 | 0 | 100 | 100 | 300 | 1 | 2 | 2 | 95 | 99 | 291 | Yes |
| 3 | F151093A4 | 0 | 20 | 40 | 40 | 100 | 220 | 5 | 15 | 40 | 40 | 95 | 215 | Yes |
| 4 | F151286A5 | 0 | 0 | 0 | 100 | 100 | 300 | 0 | 0 | 0 | 100 | 100 | 300 | Yes |
| 5 | F151288A3 | 70 | 25 | 5 | 0 | 30 | 35 | 79 | 20 | 0 | 1 | 21 | 23 | Yes |
| 6 | F151053A4 | 100 | 0 | 0 | 0 | 0 | 0 | 100 | 0 | 0 | 0 | 0 | 0 | Yes |
| 7 | F151419A5 | 40 | 40 | 20 | 0 | 60 | 80 | 40 | 50 | 10 | 0 | 60 | 70 | Yes |
| 8 | F151588A4 | 100 | 0 | 0 | 0 | 0 | 0 | 100 | 0 | 0 | 0 | 0 | 0 | Yes |
| 9 | F152115A1 | 35 | 20 | 40 | 5 | 65 | 115 | 43 | 0 | 55 | 2 | 57 | 116 | Yes |
| 10 | F152186A6 | 70 | 15 | 10 | 5 | 30 | 50 | 80 | 0 | 15 | 5 | 20 | 45 | Yes |
| 11 | F161764A4 | 100 | 0 | 0 | 0 | 0 | 0 | 100 | 0 | 0 | 0 | 0 | 0 | Yes |
| 12 | F163331A7 | 100 | 0 | 0 | 0 | 0 | 0 | 100 | 0 | 0 | 0 | 0 | 0 | Yes |
| 13 | F183408A5 | 100 | 0 | 0 | 0 | 0 | 0 | 100 | 0 | 0 | 0 | 0 | 0 | Yes |
| 14 | F183409A3 | 100 | 0 | 0 | 0 | 0 | 0 | 100 | 0 | 0 | 0 | 0 | 0 | Yes |
| 15 | F183410A4 | 100 | 0 | 0 | 0 | 0 | 0 | 100 | 0 | 0 | 0 | 0 | 0 | Yes |
| 16 | F152469A1 | 5 | 20 | 60 | 15 | 95 | 185 | 0 | 5 | 90 | 5 | 100 | 200 | Yes |
| 17 | F153482A6 | 0 | 5 | 5 | 90 | 100 | 285 | 5 | 0 | 10 | 85 | 95 | 275 | Yes |
| 18 | F160249A3 | 10 | 10 | 80 | 0 | 90 | 170 | 10 | 30 | 60 | 0 | 90 | 150 | Yes |
| 19 | F160840A4 | 10 | 45 | 20 | 30 | 95 | 175 | 10 | 50 | 0 | 40 | 90 | 170 | Yes |
| 20 | F161270A10 | 5 | 35 | 30 | 30 | 95 | 185 | 5 | 30 | 50 | 15 | 95 | 175 | Yes |
| 21 | F152459A4 | 80 | 10 | 5 | 5 | 20 | 35 | 85 | 5 | 0 | 10 | 15 | 35 | Yes |
| 22 | F152481A3 | 0 | 20 | 10 | 70 | 100 | 250 | 0 | 10 | 20 | 70 | 100 | 260 | Yes |
| 23 | F153051A6 | 0 | 0 | 0 | 100 | 100 | 300 | 0 | 0 | 0 | 100 | 100 | 300 | Yes |
| 24 | F153077A8 | 60 | 5 | 15 | 20 | 40 | 95 | 60 | 0 | 20 | 20 | 40 | 100 | Yes |
| 25 | F153286A4 | 0 | 20 | 50 | 30 | 100 | 210 | 0 | 0 | 80 | 20 | 100 | 220 | Yes |
| 26 | F150837A1 | 0 | 0 | 20 | 80 | 100 | 280 | 5 | 0 | 0 | 95 | 95 | 285 | Yes |
| 27 | F151725A1 | 10 | 30 | 40 | 20 | 90 | 170 | 0 | 60 | 20 | 20 | 100 | 160 | Yes |
| 28 | F152367A7 | 0 | 0 | 0 | 100 | 100 | 300 | 0 | 0 | 5 | 95 | 100 | 295 | Yes |
| 29 | F160053A13 | 35 | 20 | 10 | 35 | 65 | 145 | 30 | 0 | 15 | 55 | 70 | 195 | Yes |
| 30 | F160743A7 | 0 | 0 | 10 | 90 | 100 | 290 | 2 | 0 | 3 | 95 | 98 | 291 | Yes |
| 31 | F150576A4 | 0 | 0 | 10 | 90 | 100 | 290 | 0 | 0 | 0 | 100 | 100 | 300 | Yes |
| 32 | F151182A1 | 5 | 20 | 55 | 20 | 95 | 190 | 5 | 15 | 60 | 20 | 95 | 195 | Yes |
| 33 | F151461A9 | 80 | 20 | 0 | 0 | 20 | 20 | 85 | 15 | 0 | 0 | 15 | 15 | Yes |
| 34 | F151653A1 | 30 | 40 | 20 | 10 | 70 | 110 | 30 | 30 | 30 | 10 | 70 | 120 | Yes |
| 35 | F152160A5 | 5 | 5 | 90 | 0 | 95 | 185 | 5 | 0 | 95 | 0 | 95 | 190 | Yes |
| 36 | F083987A3 | 95 | 1 | 2 | 2 | 5 | 11 | 97 | 0 | 3 | 0 | 3 | 6 | Yes |
| 37 | F084245A8 | 100 | 0 | 0 | 0 | 0 | 0 | 100 | 0 | 0 | 0 | 0 | 0 | Yes |
| 38 | F102289A8 | 100 | 0 | 0 | 0 | 0 | 0 | 100 | 0 | 0 | 0 | 0 | 0 | Yes |
| 39 | F162428A6 | 30 | 20 | 20 | 30 | 70 | 150 | 30 | 0 | 65 | 5 | 70 | 145 | Yes |
| 40 | F170679A13 | 100 | 0 | 0 | 0 | 0 | 0 | 100 | 0 | 0 | 0 | 0 | 0 | Yes |
| 41 | F172071A4 | 90 | 10 | 0 | 0 | 10 | 10 | 95 | 5 | 0 | 0 | 5 | 5 | Yes |
| 42 | F084112A3 | 100 | 0 | 0 | 0 | 0 | 0 | 100 | 0 | 0 | 0 | 0 | 0 | Yes |
| 43 | F093801A3 | 100 | 0 | 0 | 0 | 0 | 0 | 100 | 0 | 0 | 0 | 0 | 0 | Yes |
| 44 | F171215A1 | 100 | 0 | 0 | 0 | 0 | 0 | 100 | 0 | 0 | 0 | 0 | 0 | Yes |
| 45 | F182108A1 | 100 | 0 | 0 | 0 | 0 | 0 | 100 | 0 | 0 | 0 | 0 | 0 | Yes |
| 46 | F185255A8 | 100 | 0 | 0 | 0 | 0 | 0 | 100 | 0 | 0 | 0 | 0 | 0 | Yes |

Consistency between pathologists for the AKR1C3 IHC assay between the two pathologists (GH and KZ) showed 100% consistency (46/46) based on AKR1C3 expression in the cytoplasm and nucleus of tumour cells and met the ≥90% of standardable limits.

To illustrate the performance characteristics of the method and these standards, representative figures of the IHC results for a subset of the assay samples were shown below (original magnification times of 20x), as shown in FIGs. 14 to 19.

### Example 5 Diagnostic kit for detecting AKR1C3

In this example, a diagnostic kit (Kit) for detecting AKR1C3 comprises:
pH 6.0 of antigen retrieval solution;
1.2 ug/ml concentration of AKR1C3 monoclonal antibody solution containing NaN₃, H⁺, Cl⁻ and tromethamine;
1.2 ug/ml concentration of secondary antibody solution containing NaN₃, H⁺, Cl⁻ and tromethamine;
blocking solution: mouse serum;
negative control reagent solution; and
instructions documenting the relevant operation methods.

The mouse AKR1C3 monoclonal antibody solution at a concentration of 1.2 ug/ml and the secondary antibody solution at a concentration of 1.2 ug/ml were obtained by diluting an antibody dilution solution, wherein the antibody dilution solution includes:
0.05 mol/L of Tris-HCl buffer (tromethamine-hydrochloric acid buffer) containing 0.1% mass ratio of polyethylene glycol or Tween, and 0.015 mol/L of sodium azide;
The secondary antibody is goat anti-mouse antibody, rabbit anti-mouse antibody, horse anti-mouse antibody or donkey anti-mouse antibody.

The negative control reagent solution is a commercially available product: FLEX Negative Control, Mouse, (Link) from DAKO Corporation.

The diagnostic kit for detecting AKR1C3, which was used together with an AKR1C3-activated anticancer prodrug, was usually used for screening patients. The use of this kit allows medical staff to perform the detection in different laboratories using a standard operating procedure (SOP) for uniform detection kits before deciding to administer the drug to a patient, so that the AKR1C3 detection results obtained with the same reagents and in the same operation can be matched to the recommended detection results for a specific cancer in the drug instructions for an AKR1C3 activated anticancer prodrug.

The specific operation methods of the kit were documented in the instructions, i.e., the specific operating conditions in the instructions above. Optionally or as a preferred embodiment, scoring values for the IHC staining assay using AKR1C3-activated anticancer prodrugs for different cancer (tumour) types were also given in these instructions. For example, as for gastric cancer, the score (e.g. H score) for gastric cancer tissue specimen from a certain patient was 209 by using the above-mentioned kit to detect and score, while it was statistically obtained that the score of IHC staining detection method using AKR1C3-activated anticancer prodrug in a gastric cancer patient cannot be less than 165, and thus the doctor can prescribe AKR1C3-activated anticancer prodrug for this patient. As another example, for esophageal cancer, the score for esophageal cancer tissue specimen from a certain patient was 105 by using the above-mentioned kit to detect and score, while it was statistically obtained that the score of IHC staining detection method using AKR1C3-activated anticancer prodrug in a esophageal cancer patient cannot be less than 115, and thus the doctor cannot prescribe AKR1C3-activated anticancer prodrug for this patient.

Example 6 Use of AKR1C3 detection method and diagnostic kit for detecting AKR1C3 in the treatment of cancer, tumour or cell proliferation disease

The scoring value (e.g. H score) of isolated formalin-fixed paraffin-embedded human tissue specimen from a patient with gastric cancer detected by the AKR1C3 detection method established in Example 1 or the diagnostic kit for detecting AKR1C3 in Example 5 was 209, which was greater than the predetermined scoring value of 165;

AKR1C3-activated anticancer drugs were administered to this gastric cancer patient.

As verified by existing trials, AKR1C3-activated anticancer drugs selected from the following structures may have the best therapeutic effect: 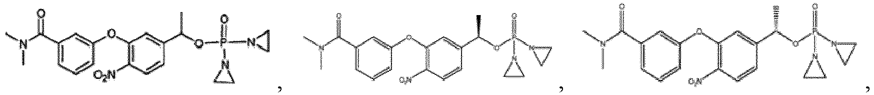 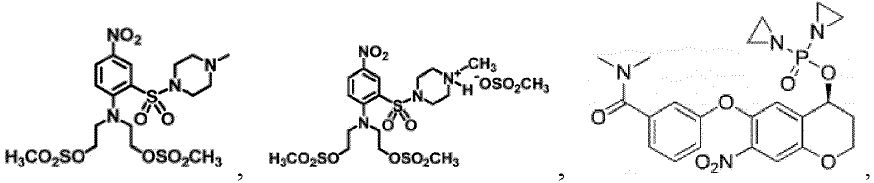 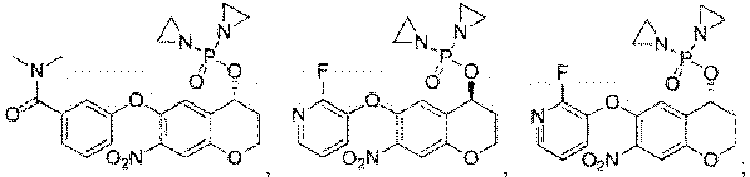 or 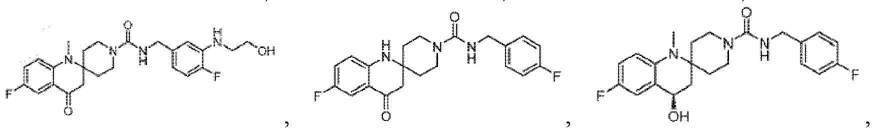 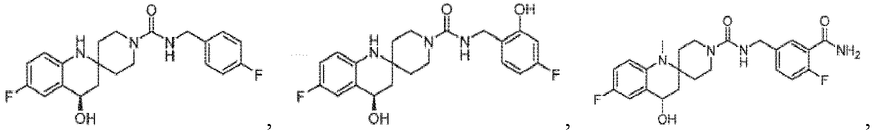 a pharmaceutically acceptable salt or isomer thereof.

## Claims

1. An AKR1C3 detection method, wherein the AKR1C3 expression levels in isolated formalin-fixed paraffin-embedded human tissue specimen is detected by using immunohistochemical staining method, comprising the following steps:
a) antigen retrieval
performing antigen retrieval by heating the formalin-fixed paraffin-embedded human tissue specimen at 90~115°C for 17-30 min in the presence of an antigen retrieval solution;
b) primary antibody incubation
mixing the antigen-retrieved formalin-fixed paraffin-embedded human tissue specimen with a 0.5~5.0 µg/ml concentration of AKR1C3 monoclonal antibody solution for incubation for 25-700 min;
c) secondary antibody incubation
mixing the primary antibody-incubated formalin-fixed paraffin-embedded human tissue specimen with a 0.5~5.0 µg/ml concentration of secondary antibody solution and incubating for 25-700 min.

2. The AKR1C3 detection method according to claim 1, wherein in the antigen retrieval of step a), the antigen retrieval solution has a pH of 2.0 - 9.0;
more preferably, the antigen retrieval solution has a pH of 6.0 ~ 9.0;
even more preferably, the antigen retrieval solution has a pH of 6.0.

3. The AKR1C3 detection method according to claim 1 or 2, wherein in the antigen retrieval of step a), the antigen retrieval solution includes sodium citrate antigen retrieval solution or EDTA antigen retrieval solution.

4. The AKR1C3 detection method according to any one of claims 1-3, wherein in the antigen retrieval of step a), the formalin-fixed paraffin-embedded human tissue specimen is heated at 92-102 °C for 18-25 min;
more preferably, the formalin-fixed paraffin-embedded human tissue specimen is heated at 97 °C for 20 min.

5. The AKR1C3 detection method according to any one of claims 1-4, wherein in the primary antibody incubation of step b), the AKR1C3 monoclonal antibody solution has a concentration of 1.0~3.0 µg/ml;
more preferably, the AKR1C3 monoclonal antibody solution has a concentration of 1.2 µg /ml;
and/or, in the secondary antibody incubation of step c), the secondary antibody solution has a concentration of 1.0~3.0 µg/ml;
more preferably, the secondary antibody solution has a concentration of 1.2 µg/ml.

6. The AKR1C3 detection method according to any one of claims 1-5, wherein the AKR1C3 monoclonal antibody solution and the secondary antibody solution both contain NaN₃, H⁺, Cl⁻ and tromethamine.

7. The AKR1C3 detection method according to claim 6, wherein the AKR1C3 monoclonal antibody solution and the secondary antibody solution are obtained by diluting with an antibody dilution buffer, wherein the antibody dilution buffer comprises the following components:
0.02~0.08 mol/L of Tris-HCl buffer,
containing 0.05~0.15% mass concentration of polyethylene glycol or Tween, and
0.010~0.020 mol/L of sodium azide;
more preferably, the antibody dilution buffer comprises the following components:
0.05 mol/L of Tris-HCl buffer,
containing 0.1% mass concentration of polyethylene glycol or Tween, and
0.015 mol/L of sodium azide.

8. The AKR1C3 detection method according to any one of claims 1-7, wherein in the primary antibody incubation of step b), the antigen-retrieved formalin-fixed paraffin-embedded human tissue specimen is incubated with the AKR1C3 monoclonal antibody solution for 30-45 min;
more preferably, the antigen-retrieved formalin-fixed paraffin-embedded human tissue specimen is incubated with the AKR1C3 monoclonal antibody solution for 45 min.

9. The AKR1C3 detection method according to any one of claims 1-8, wherein in the secondary antibody incubation of step c), the primary antibody-incubated formalin-fixed paraffin-embedded human tissue specimen is incubated with the secondary antibody solution for 30~45 min;
more preferably, the primary antibody-incubated formalin-fixed paraffin-embedded human tissue specimen is incubated with the secondary antibody solution for 30 min.

10. The AKR1C3 detection method according to any one of claims 1-9, wherein in the primary antibody incubation of step b), the AKR1C3 monoclonal antibody is a mouse monoclonal antibody;
and/or, in the secondary antibody incubation of step c), the secondary antibody is a goat anti-mouse antibody, a rabbit anti-mouse antibody, a horse anti-mouse antibody or a donkey anti-mouse antibody.

11. The AKR1C3 detection method according to any one of claims 1-10, wherein after the secondary antibody incubation of step c), further comprising:
d) staining and sealing
staining the formalin-fixed paraffin-embedded human tissue specimen using hematoxylin, and performing the dehydration and sealing of specimen after staining.

12. The AKR1C3 detection method according to any one of claims 1-11, wherein before the antigen retrieval of step a), further comprising:
a1) dewaxing and rehydration
dewaxing the formalin-fixed paraffin-embedded human tissue specimen using an organic solvent, and washing the dewaxed specimen sequentially using alcohols containing different water contents, and finally washing with water;
preferably, the organic solvent is acetone, toluene or xylene; more preferably, the organic solvent is xylene;
and/or, preferably, the alcohol is ethanol or methanol; more preferably, the alcohol is ethanol;
and/or, preferably, the dewaxed specimen is first washed with anhydrous ethanol and then washed with ethanol having a volume fraction of 90~97%.

13. The AKR1C3 detection method according to any one of claims 1-12, wherein between the antigen retrieval of step a) and the primary antibody incubation of step b), further comprising:
b1) blocking non-specific antigen
co-incubating the antigen-retrieved formalin-fixed paraffin-embedded human tissue specimen with a blocking solution to block a non-specific antigen;
preferably, the blocking solution is a serum of an animal from which the AKR1C3 monoclonal antibody is derived;
more preferably, the blocking solution is mouse serum.

14. The AKR1C3 detection method according to any one of claims 1-13, wherein the formalin-fixed paraffin-embedded human tissue specimen is breast cancer tissue specimen, colorectal cancer tissue specimen, esophageal cancer tissue specimen, gastric cancer tissue specimen, hepatocellular carcinoma tissue specimen, non-small cell lung cancer tissue specimen, prostate cancer tissue specimen, renal cell carcinoma specimen, peripheral T-cell lymphoma specimen or nodular NK/T-cell lymphoma specimen.

15. A diagnostic kit for detecting AKR1C3, comprising:
antigen retrieval solution;
a 0.5~5.0 µg/ml concentration of AKR1C3 monoclonal antibody solution;
a 0.5~5.0 µg/ml concentration of secondary antibody solution.

16. The diagnostic kit for detecting AKR1C3 according to claim 15, wherein the antigen retrieval solution has a pH of 2.0 ~ 9.0;
more preferably, the antigen retrieval solution has a pH of 6.0 ~ 9.0;
even more preferably, the antigen retrieval solution has a pH of 6.0.

17. The diagnostic kit for detecting AKR1C3 according to claim 15 or 16, wherein the antigen retrieval solution includes sodium citrate antigen retrieval solution or EDTA antigen retrieval solution.

18. The diagnostic kit for detecting AKR1C3 according to any one of claims 15-17, wherein the AKR1C3 monoclonal antibody solution has a concentration of 1.0~3.0 µg/ml;
more preferably, the AKR1C3 monoclonal antibody solution has a concentration of 1.2 µg /ml;
and/or, the secondary antibody solution has a concentration of 1.0~3.0 µg/ml;
more preferably, the secondary antibody solution has a concentration of 1.2 µg/ml.

19. The diagnostic kit for detecting AKR1C3 according to any one of claims 15-18, wherein the AKR1C3 monoclonal antibody solution and the secondary antibody solution both contain NaN₃, H⁺, Cl⁻ and tromethamine.

20. The diagnostic kit for detecting AKR1C3 according to claim 19, wherein the AKR1C3 monoclonal antibody solution and the secondary antibody solution are obtained by diluting with an antibody dilution buffer, wherein the antibody dilution buffer comprises the following components:
0.02~0.08 mol/L of Tris-HCl buffer,
containing 0.05~0.15% mass concentration of polyethylene glycol or Tween, and
0.010~0.020 mol/L of sodium azide;
more preferably, the antibody dilution buffer comprises the following components:
0.05 mol/L of Tris-HCl buffer,
containing 0.1% mass concentration of polyethylene glycol or Tween, and
0.015 mol/L of sodium azide.

21. The diagnostic kit for detecting AKR1C3 according to any one of claims 15-20, wherein the AKR1C3 monoclonal antibody is a mouse monoclonal antibody;
and/or, the secondary antibody is a goat anti-mouse antibody, a rabbit anti-mouse antibody, a horse anti-mouse antibody or a donkey anti-mouse antibody.

22. The diagnostic kit for detecting AKR1C3 according to any one of claims 15-21, further comprising:
blocking solution, preferably, the blocking solution is a serum of an animal from which the AKR1C3 monoclonal antibody is derived;
more preferably, the blocking solution is mouse serum.

23. The diagnostic kit for detecting AKR1C3 according to any one of claims 15-22, further comprising:
negative control reagent solution; and
instructions.

24. Use of the diagnostic kit for detecting AKR1C3 according to any one of claims 15-23 in the preparation of drugs for the treatment of cancer, tumor or cell proliferative disease.

25. The use according to claim 24, comprising the following steps:
obtaining AKR1C3 expression levels in isolated formalin-fixed paraffin-embedded human tissue specimen from a patient using the diagnostic kit for detecting AKR1C3;
administering AKR1C3-activated anticancer drug to the patient whose AKR1C3 expression levels are greater than or equal to predetermined expression levels.

26. The use according to claim 25, wherein the AKR1C3-activated anticancer drug meets at least one of the following definitions:
A. in the presence of an AKR1C3 inhibitor, the detected inhibition effect of a compound on the proliferation of cancer cells is less than that in the absence of an AKR1C3 inhibitor;
B. the inhibition effect of a compound on the proliferation of cancer cells with different expression levels of AKR1C3 enzyme is significantly different, and the inhibition effect on the proliferation of cancer cells with high AKR1C3 enzyme expression is much greater than that of cancer cells with low AKR1C3 enzyme expression;
C. when the inhibition effect of certain aldehyde-ketone compound containing carbon-oxygen double bond on the proliferation of cancer cells with different expression levels of AKR1C3 enzyme is significantly different, and the inhibition effect on the proliferation of cancer cells with high AKR1C3 enzyme expression is much greater than that of cancer cells with low AKR1C3 enzyme expression, and the difference among the inhibition effects of the corresponding alcohol compound containing hydroxyl group on the proliferation of cancer cells with different expression levels of AKR1C3 enzyme is small or similar, then the aldehyde-ketone compound containing carbon-oxygen double bond is AKR1C3-activated anticancer drug, and the corresponding alcohol compound containing hydroxyl group is parent drug.

27. The use according to claim 25 or 26, wherein the AKR1C3-activated anticancer drug is selected from the compounds of the following structures: 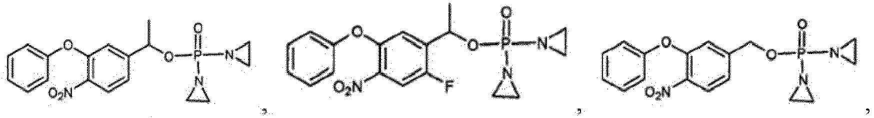 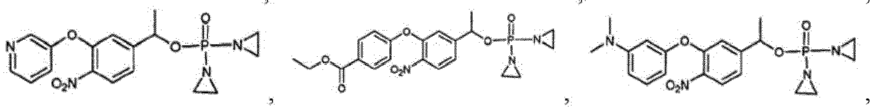 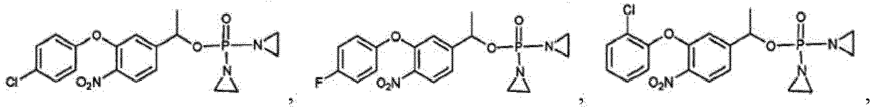 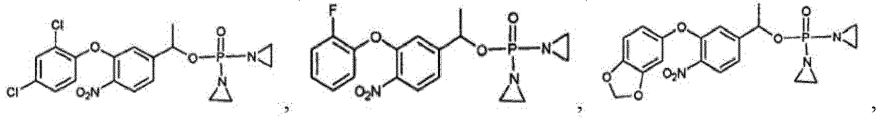 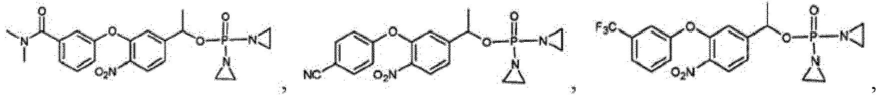 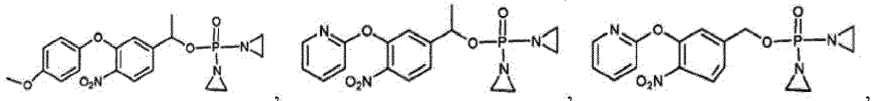 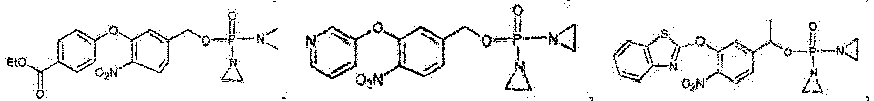 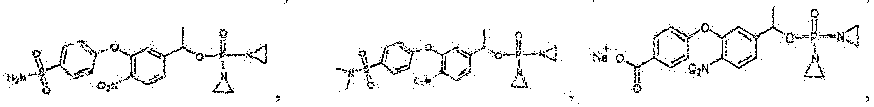 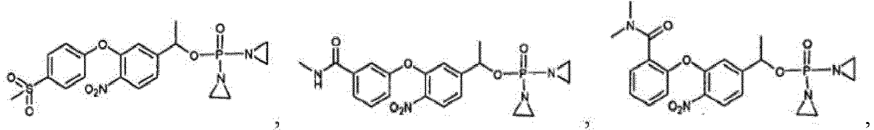 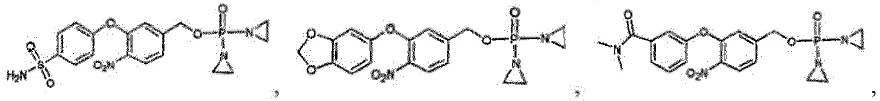 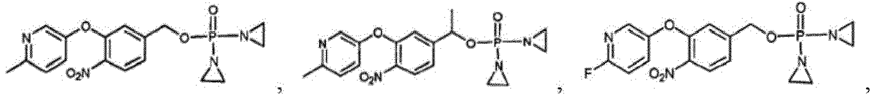 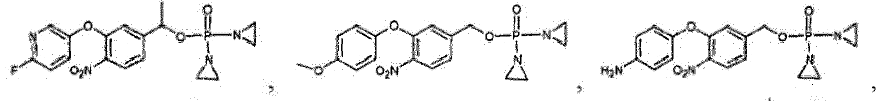 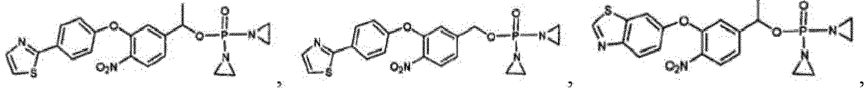 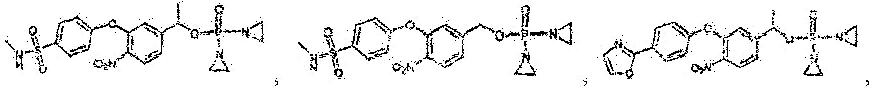 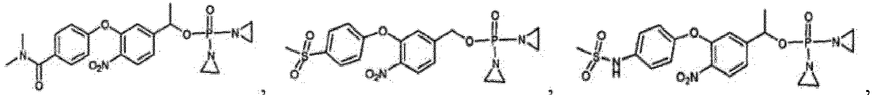 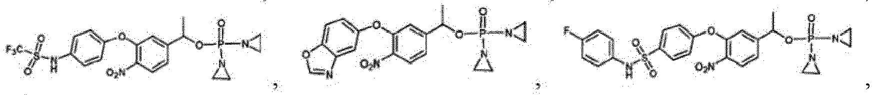 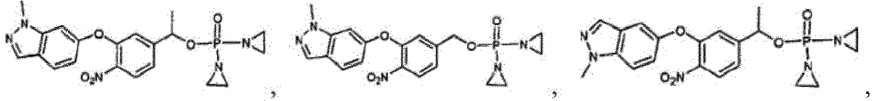 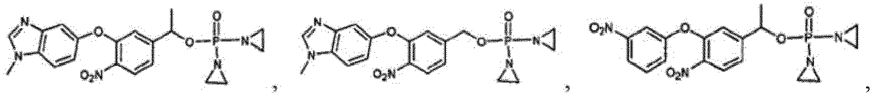 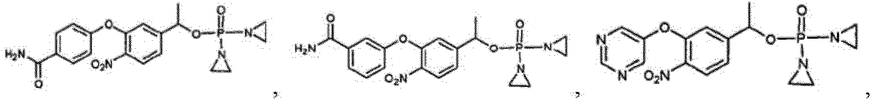 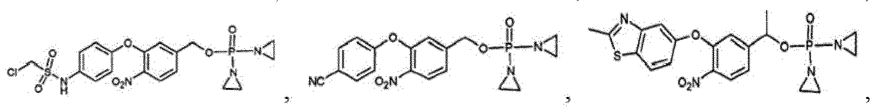 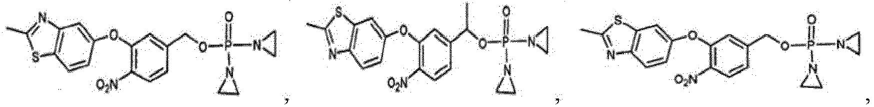 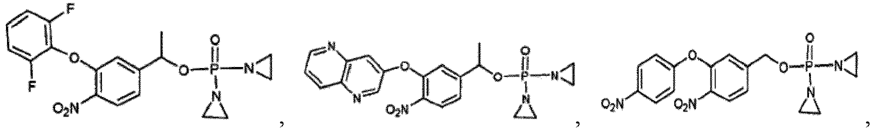 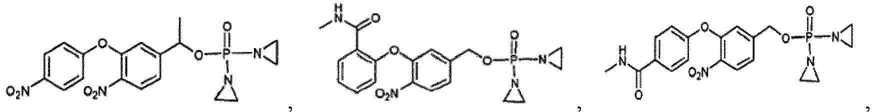 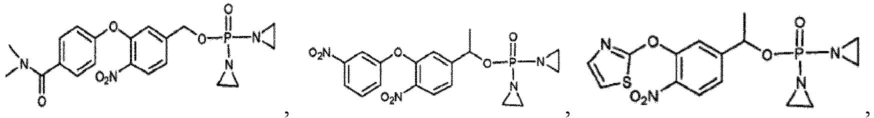 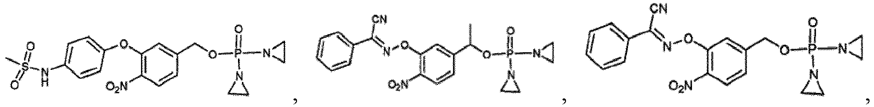 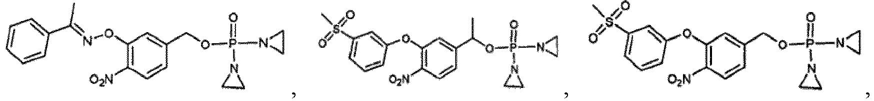 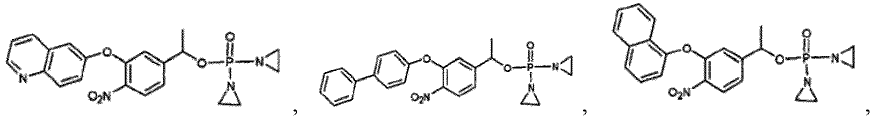 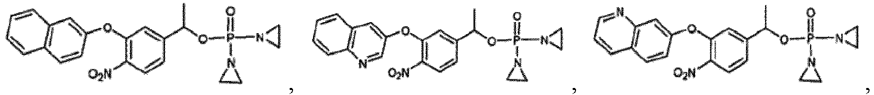 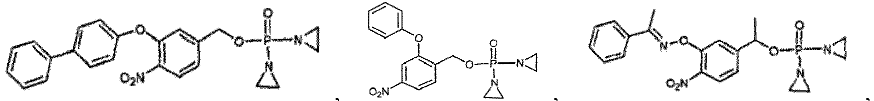 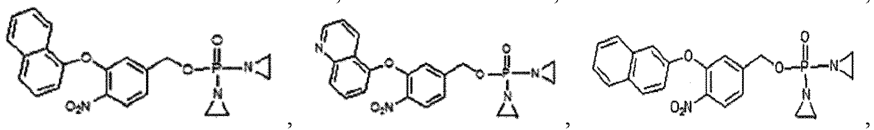 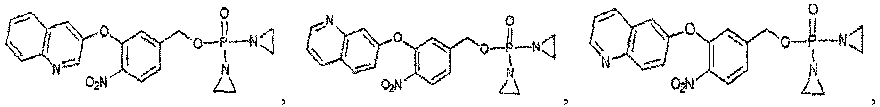 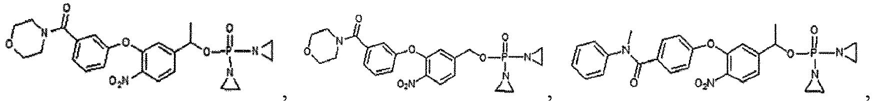 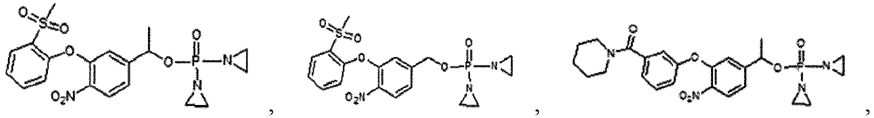 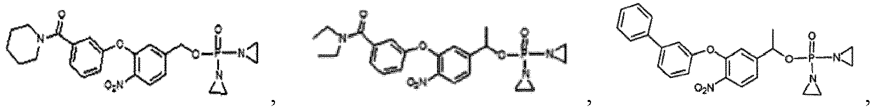 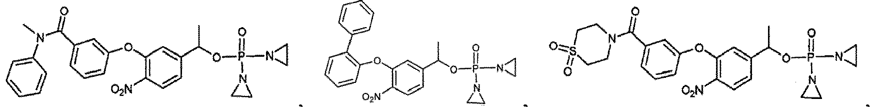 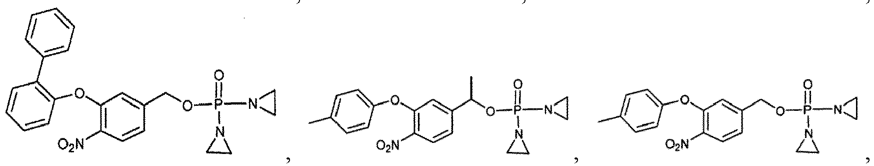 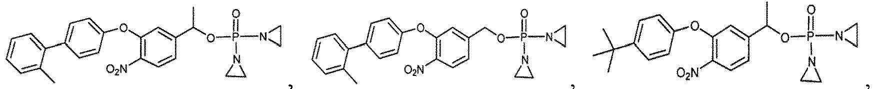 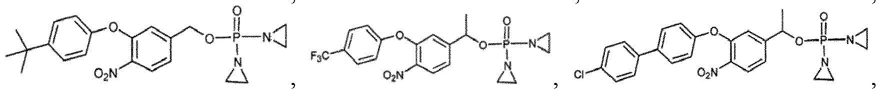 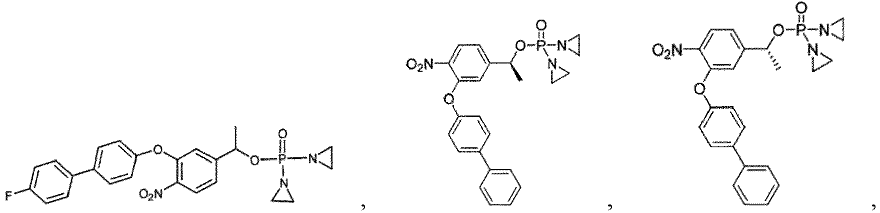 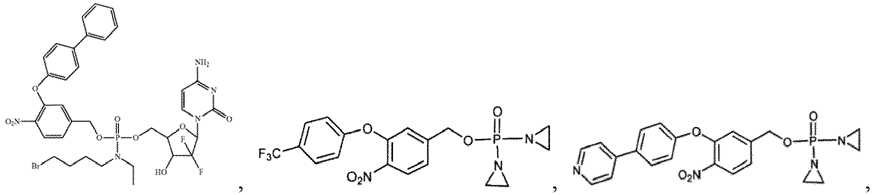 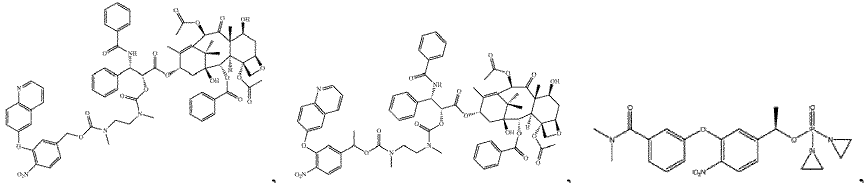 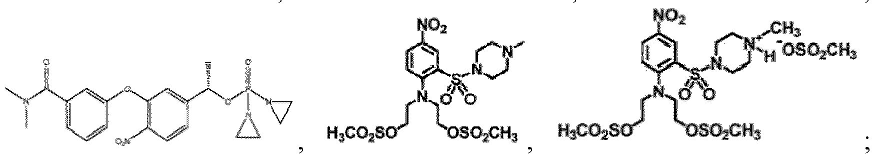 or 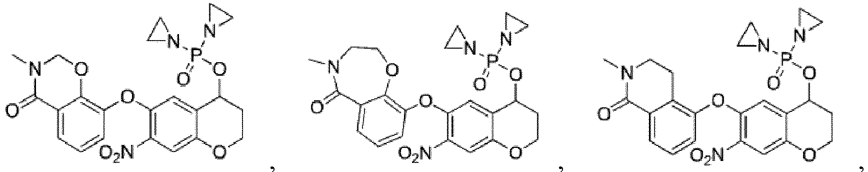 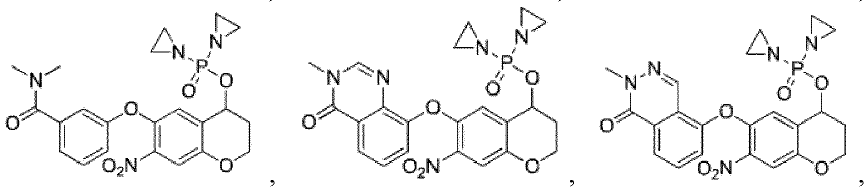 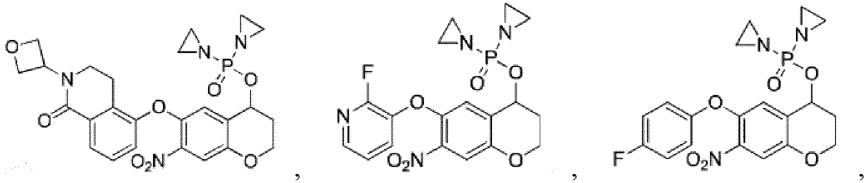 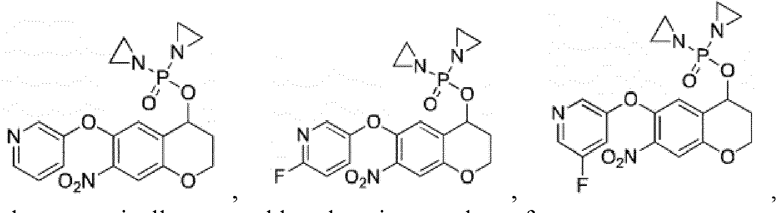 or a pharmaceutically acceptable salt or isomer thereof; or 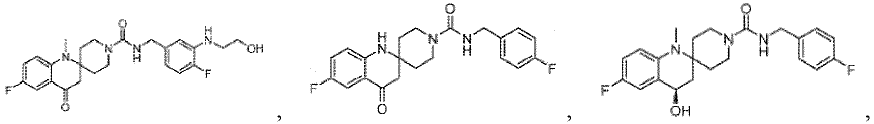 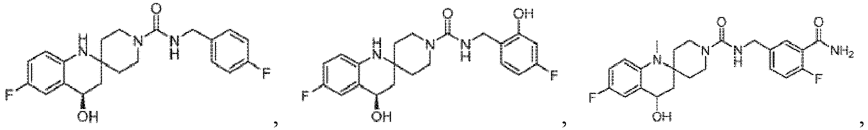 or a pharmaceutically acceptable salt or isomer thereof;
preferably, the AKR1C3-activated anticancer drug is selected from the compounds of the following structures: 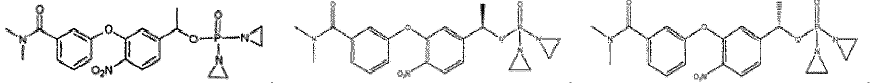 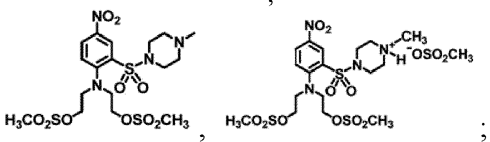 or 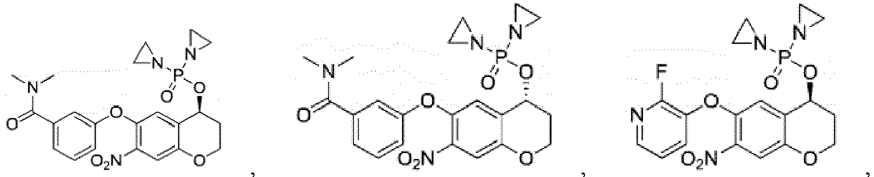 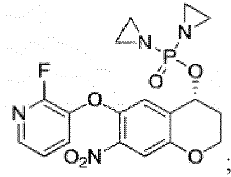 or 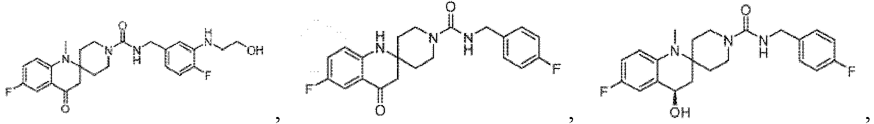 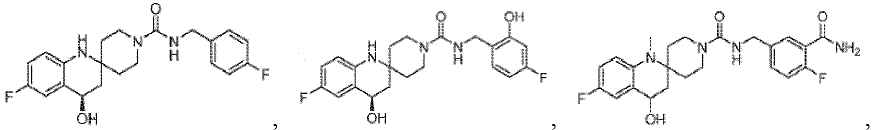 or a pharmaceutically acceptable salt or isomer thereof.

28. The use according to any one of claims 24-27, wherein the cancer, tumor or cell proliferative disease comprises:
lung cancer, non-small cell lung cancer, liver cancer, pancreatic cancer, breast cancer, gastric cancer, bone cancer, esophageal cancer, mastocarcinoma, prostate cancer, testicular cancer, colon cancer, ovarian cancer, bladder cancer, cervical cancer, hepatocellular carcinoma, melanoma, squamous cell carcinoma, basal cell carcinoma, adenocarcinoma, sweat gland carcinoma, sebaceous gland carcinoma, papillary carcinoma, papillary adenocarcinoma, renal cell carcinoma, cystic adenocarcinoma, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, peripheral T-cell lymphoma, nodular NK/T-cell lymphoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, chronic disease cells, polycythemia, lymphoma, endometrial cancer, glioma, colorectal cancer, thyroid cancer, urothelial cancer or multiple myeloma;
preferably, the cancer, tumour or cell proliferative disease comprises: ovarian cancer, cervical cancer, pancreatic cancer, breast cancer, colorectal cancer, esophageal cancer, gastric cancer, hepatocellular carcinoma, non-small cell lung cancer, prostate cancer, renal cell carcinoma, peripheral T-cell lymphoma or nodular NK/T-cell lymphoma.
